# EUROPEAN PATENT APPLICATION

(11) **EP 4 410 186 A1**
(43) Date of publication of application: **07.08.2024**
(21) Application number: 24152578.1
(22) Date of filing: 18.01.2024
(51) Int. Cl.: A61B 5/00, A61B 5/25

(54) **BIOPOTENTIAL MEASUREMENT SYSTEM WITH ELECTRODE PREHEATING UNIT**

(30) Priority: 31.01.2023 US 202318162519
(71) Applicant: Meta Platforms Technologies, LLC, Menlo Park, CA 94025 (US)
(72) Inventor: Yokus, Murat, Menlo Park, 94025 (US); Alpkilic, Ahmet, Menlo Park, 94025 (US); Agcayazi, Muhammed, Menlo Park, 94025 (US); Tolosa, Vanessa, Menlo Park, 94025 (US)
(74) Representative: Murgitroyd & Company

(57) **Abstract**

A disclosed biopotential measurement system may include a wearable device having 1) at least one bioelectrode positioned to abut a user's skin when the wearable device is worn by the user and 2) biosignal-acquiring circuitry that captures a biosignal from the user's body via the at least one bioelectrode. Measurement performance of the at least one bioelectrode may be substantially optimized when a temperature of the at least one bioelectrode is at or above a threshold temperature. Various other methods, systems, and computer-readable media are also disclosed.

## Description

The present invention relates to a biopotential measurement system and in particular to such a system that may include a wearable device.

In this connection, obtaining consistent high-quality biosignals using conventional electrodes and conventional signal processing techniques is challenging, in part due to impedance mismatches at the interface between a user's skin and the electrodes.

### SUMMARY

According to an aspect of the present invention, there is provided a biopotential measurement system comprising: a wearable device comprising: at least one bioelectrode positioned to abut a user's skin when the wearable device is worn by the user; and biosignal-acquiring circuitry that captures a biosignal from the user's body via the at least one bioelectrode, wherein measurement performance of the at least one bioelectrode is substantially optimized when a temperature of the at least one bioelectrode is at or above a threshold temperature.

Optionally, the biopotential measurement system further comprises a temperature sensor for measuring a temperature of at least one of the at least one bioelectrode or a portion of the user's skin.

Optionally, the threshold temperature comprises a temperature of approximately 30 °C or more.

Optionally, a skin-electrode impedance of the at least one bioelectrode is approximately 0.6 MΩ or less when the temperature of the at least one bioelectrode is at or above the threshold temperature.

Optionally, the wearable device is configured to extend around at least a portion of the user's arm or head; and the at least one bioelectrode is positioned at an inner surface portion of the wearable device.

Optionally, the biopotential measurement system further comprises an indicator that provides notification to the user of whether the bioelectrode is at or above the threshold temperature.

Optionally, the at least one bioelectrode comprises a metal material.

Optionally, the biopotential measurement system further comprises a heating subsystem that heats the at least one bioelectrode.

Optionally, the heating subsystem preheats the at least one bioelectrode to the temperature at or above the threshold temperature when the wearable device is not worn by the user.

Optionally, the heating subsystem is located in the wearable device.

Optionally, the biopotential measurement system further comprises a charging device for providing power to the wearable device, wherein the heating subsystem is located in the charging device.

Optionally, the charging device comprises a charging dock; and the heating subsystem heats the at least one bioelectrode when the wearable device is disposed on the charging dock.

Optionally, the heating subsystem comprises a heating element.

Optionally, the heating element is disposed in close proximity to the bioelectrode.

Optionally, the heating element comprises at least one of a conductive heating element or a radiant heating element.

Optionally, the at least one bioelectrode comprises a plurality of electrodes disposed at different locations.

According to a further aspect of the present invention, there is provided a wearable device comprising: at least one bioelectrode positioned to abut a user's skin when the wearable device is worn by the user; biosignal-acquiring circuitry that captures a biosignal from the user's body via the at least one bioelectrode; and a heating subsystem that heats the at least one bioelectrode.

According to a further aspect of the present invention there is provided a method comprising: heating at least one bioelectrode of a wearable device such that a temperature of the at least one bioelectrode is at or above a threshold temperature; positioning the at least one bioelectrode abutting a user's skin when the wearable device is worn by the user; and capturing a biosignal from the user's body via the at least one bioelectrode.

Optionally, the threshold temperature comprises a temperature of approximately 30 °C or more.

Optionally, the method further comprises heating the at least one bioelectrode prior to positioning the at least one bioelectrode abutting the user's skin.

### BRIEF DESCRIPTION OF THE DRAWINGS

The accompanying drawings illustrate a number of exemplary embodiments and are a part of the specification. Together with the following description, these drawings demonstrate and explain various principles of the present disclosure.
FIG. 1A is a perspective view of an exemplary wearable system including a wearable device configured to be worn around a user's lower arm or wrist, according to some embodiments;
FIG. 1B is a cross-sectional view of the wearable system illustrated in FIG 1A, according to some embodiments;
FIG. 2 is a schematic diagram of components of an exemplary wearable system, in accordance with some embodiments;
FIG. 3 is a schematic diagram of an exemplary wearable system that includes a wearable device with a built-in heating subsystem, according to some embodiments;
FIG. 4 is a schematic diagram of an exemplary wearable system that includes a wearable device and a charging device having a heating subsystem, according to some embodiments;
FIG. 5A is a graph showing initial electrode temperatures and final electrode temperatures after being worn by a user for a period of 30 minutes, in accordance with some embodiments;
FIG. 5B is a graph showing differences between the corresponding initial and final electrode temperatures shown in FIG. 5A, in accordance with some embodiments;
FIG. 6A is a graph showing initial impedance values for the electrodes at the initial electrode temperature values shown in FIG. 5A, in accordance with some embodiments;
FIG. 6B is a graph showing the initial impedance values of FIG. 6A versus the temperature differences shown in FIG. 5B for each of the electrodes, in accordance with some embodiments;
FIG. 7A is a graph showing settling times for each of the electrodes described in reference to FIGS. 5A-6B, in accordance with some embodiments;
FIG. 7B is a graph showing final impedance values for the electrodes versus initial electrode temperature values shown in FIG. 7A, in accordance with some embodiments;
FIG. 8 is a graph showing phases after 30 minutes of skin contact for each of the electrodes described in reference to FIGS. 5A-7B, in accordance with some embodiments;
FIG. 9 is a flow diagram of an exemplary method for operating a biopotential measurement system having an electrode preheating unit, in accordance with some embodiments;
FIG. 10 is an illustration of exemplary wearable watch device that may be used in connection with embodiments of this disclosure;
FIG. 11 is an illustration of a user with various wearable devices in connection with embodiments of this disclosure;
FIG. 12 is an illustration of exemplary augmented-reality glasses that may be used in connection with embodiments of this disclosure;
FIG. 13 is an illustration of an exemplary virtual-reality headset that may be used in connection with embodiments of this disclosure;
FIG. 14 is an illustration of exemplary haptic devices that may be used in connection with embodiments of this disclosure;
FIG. 15 is an illustration of an exemplary virtual-reality environment according to embodiments of this disclosure;
FIG. 16 is an illustration of an exemplary augmented-reality environment according to embodiments of this disclosure; and
FIGS. 17A and 17B are illustrations of an exemplary schematic diagram with internal components of a wearable system.

Throughout the drawings, identical reference characters and descriptions indicate similar, but not necessarily identical, elements. While the exemplary embodiments described herein are susceptible to various modifications and alternative forms, specific embodiments have been shown by way of example in the drawings and will be described in detail herein. However, the exemplary embodiments described herein are not intended to be limited to the particular forms disclosed. Rather, the present disclosure covers all modifications, equivalents, and alternatives falling within the scope of the appended claims.

### DETAILED DESCRIPTION OF EXEMPLARY EMBODIMENTS

Obtaining consistent high-quality biosignals using conventional electrodes and conventional signal processing techniques is challenging, in part due to impedance mismatches at the interface between a user's skin and the electrodes. For applications that require near real-time analysis of biosignals, the acquisition of consistent high-quality signals is helpful to be able to iterate quickly on the recorded data. Poor electromechanical coupling of electrodes and the human body may greatly increase signal noise and may make captured biological signals unusable for analysis. Some conventional techniques for electromechanically coupling electrodes and the human body include the use of wet electrodes or the use of dry electrodes in combination with skin preparations (e.g., shaving, sanding, or hydrating with cream). Currently, wet type and gelled electrodes present low skin electrode impedance values. However, their implementation in wearables, such as wristbands, is often limited in many situations due to skin irritation, drying of gels, long-term use, and user comfort. Other conventional techniques for electromechanically coupling electrodes and the human body include using adhesives or pressing the electrodes firmly against users' skin, which may be inconvenient and/or cause discomfort for the users.

Commonly used dry electrodes tend to have considerable variability in contact quality caused by variations at the electrode-skin interface. Electrodes may perform particularly poorly during an initial period after the electrodes first make contact with skin. Contact quality of dry electrodes with skin may be measured by determining skin-electrode electrical impedance. When a wrist-worn device with dry electrodes is donned, the initial recorded value of the skin-electrode impedance is typically high and drops over time as the electrodes reach equilibrium with the skin. Due to the high initial impedance of dry electrodes, the signal-to-noise ratio of the acquired signal and the efficiency of algorithms that use those signals as the raw data may be negatively impacted within the first few minutes of use. Accordingly, wearable devices may not provide accurate biopotential data around the time that users initially begin wearing the devices. From a consumer device perspective, this initial low performance of the device may lead to a poor first impression from the user and eventually may lead to user abandonment. Therefore, low starting impedance values are desired for the improved signal quality.

The present disclosure is generally directed to biopotential measurement systems and devices that include electrodes that are preheated with electrode preheating units. The disclosure details various exemplary designs and configurations of systems, particularly wearable systems, that have electrodes for biological-signal measurement. The disclosed systems and devices may include heating subsystems that preheat electrodes prior to being worn by a user. Preheating the electrodes to a specified temperature or range may significantly improve electrode performance during an initial period after a user begins wearing a device. Such improvements may enable the capture of more accurate biosignal data within a shortened window of time. Initial impedance values between an electrode and a user's skin may be reduced by such preheating, resulting in greatly reduced signal-to-noise ratios and improved signal quality. Additionally, preheating the electrodes may warm the electrodes to a temperature closer to the user's skin temperature, thereby improving user comfort when the device is first worn. The disclosed systems and methods may eliminate the need to use invasive or uncomfortable skin preparations in combination with dry electrodes.

Features from any of the embodiments described herein may be used in combination with one another in accordance with the general principles described herein. These and other embodiments, features, and advantages will be more fully understood upon reading the following detailed description in conjunction with the accompanying drawings and claims.

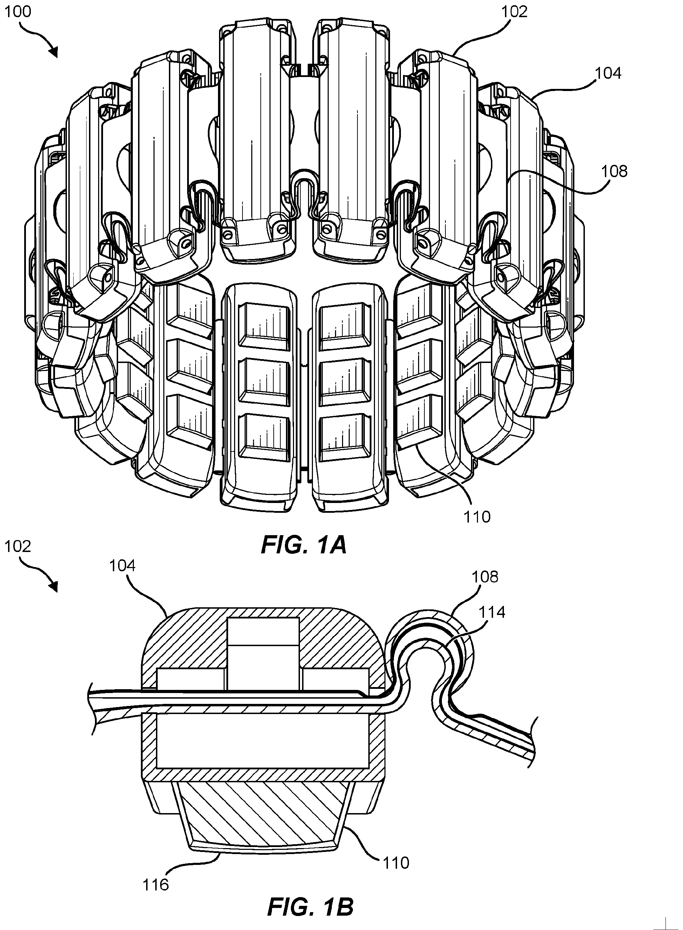

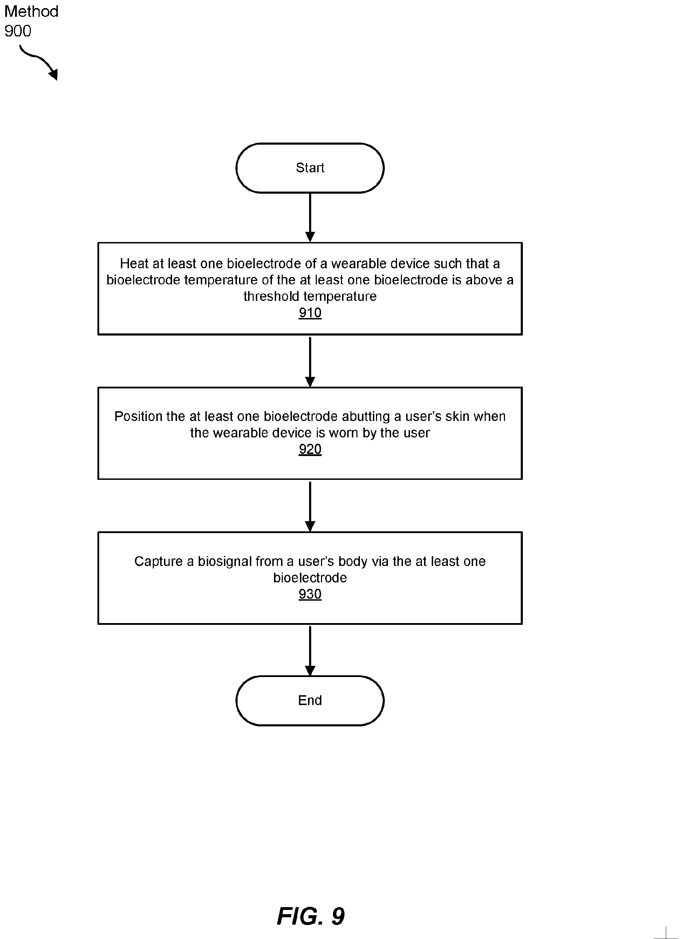

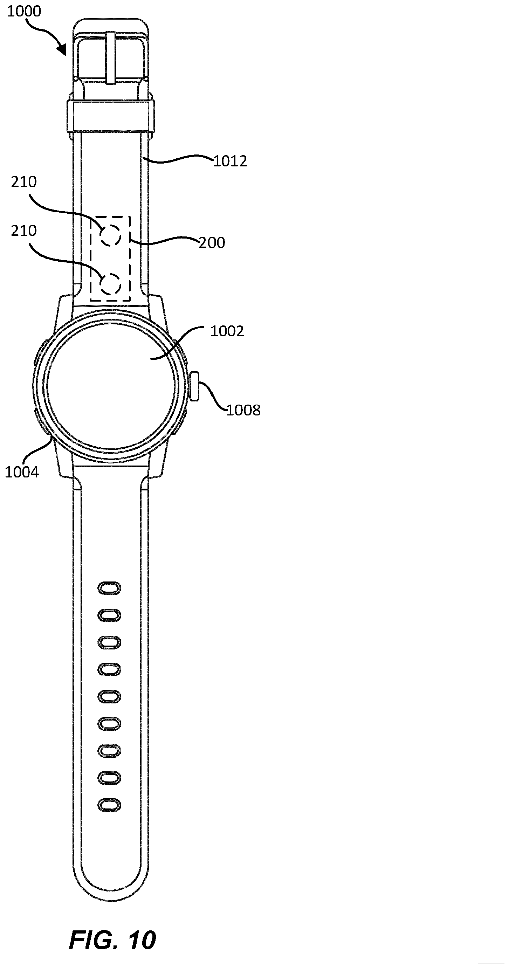

The following will provide, with reference to FIGS. 1A-8, detailed descriptions of exemplary biopotential measurement systems and devices. The discussion corresponding to FIG. 9 will provide examples of methods for operating the systems and devices presented herein. The descriptions corresponding to FIGS. 10-17B will provide examples of various systems and devices implementing embodiments presented herein.

FIG. 1A illustrates an exemplary wearable system 100 that includes a wearable device 102 configured to be worn, for example, around a user's lower arm or wrist. In this example, wearable device 102 may include sixteen neuromuscular sensors 104 (e.g., EMG sensors, etc.) arranged circumferentially around an elastic band 108. However, any suitable number of neuromuscular sensors 104 may be used. Each neuromuscular sensor 104 may include one or more bioelectrodes 110 disposed along an interior portion of the wearable device 102 such that the bioelectrodes 110 abut the user's skin when wearable device 102 is worn. Bioelectrodes 110 may be formed of an electrically conductive material, such as a metal, conductive polymer, composite, and/or any other suitable material or combination of materials (e.g., bioelectrodes 110 may each have a layered structure that includes layers of two or more different materials).

Bioelectrodes 110 may be oriented and configured to contact a user's skin when system 100 is worn by a user. The number and arrangement of neuromuscular sensors 104 and bioelectrodes 110 may depend on the particular application for which wearable device 102 is used. For example, a wearable armband or wristband can be used to generate control information for controlling an augmented reality system, a robot, controlling a vehicle, scrolling through text, controlling a virtual avatar, 4 suitable control task. As shown, neuromuscular sensors 104 may be coupled together using flexible electronics incorporated into the wireless device.

FIG. 1B illustrates a cross-sectional view through one of neuromuscular sensors 104 of wearable device 102 shown in FIG. 1A. As shown in FIG. 1B, elastic band 108 may be coupled to neuromuscular sensors 104, and in some examples, may extend through at least a portion of each of neuromuscular sensors 104. In various examples, wiring 114 may electrically connect neuromuscular sensors 104 to each other and/or to other electronic components of wearable system 100. Bioelectrodes 110 of neuromuscular sensors 104 may each include an electrode surface 116 that is shaped and configured to contact or otherwise abut a portion of a user's skin. Electrode surface 116 of a bioelectrode 110 may have a flat, uneven, patterned, or any other suitable surface shape, without limitation.

In some embodiments, the output of one or more of the sensing components can be optionally processed using hardware signal processing circuitry (e.g., to perform amplification, filtering, and/or rectification). In other embodiments, at least some signal processing of the output of the sensing components can be performed in software. Thus, signal processing of signals sampled by the sensors can be performed in hardware, software, or by any suitable combination of hardware and software, as aspects of the technology described herein are not limited in this respect.

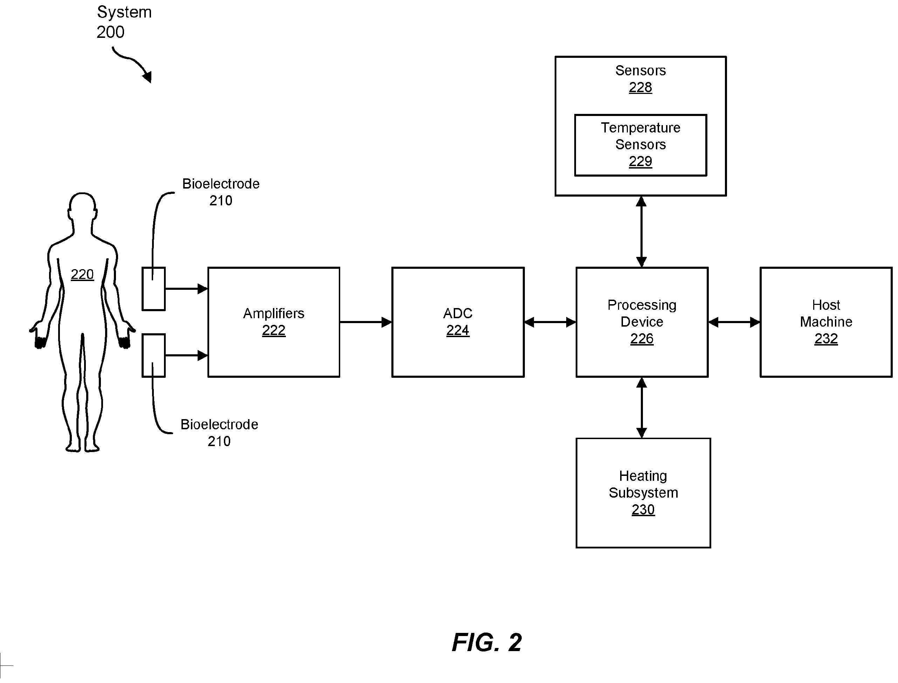

FIG. 2 schematically illustrates components of a biosignal system 200 in accordance with some embodiments. System 200 includes a pair of bioelectrodes 210 configured to register or measure a biosignal, such as an electrooculography (EOG) signal, an electromyography (EMG) signal, an electroencephalography (EEG) signal, an electrocardiography (ECG) signal, and/or any other suitable signal generated by the body of a user 220 (e.g., for electrophysiological monitoring or stimulation). In some embodiments, bioelectrodes 210 may be arranged as a portion of a wearable device configured to be worn on or around part of a user's body. For example, in one nonlimiting example, a plurality of composite bioelectrodes including bioelectrodes 210 may be arranged circumferentially around an adjustable and/or elastic band such as a wristband or armband configured to be worn around a user's wrist or arm. Additionally or alternatively, at least some of bioelectrodes 210 may be arranged on a wearable patch configured to be affixed to or placed in contact with a portion of the body of user 220. It should be appreciated that any suitable number of composite bioelectrodes may be used, and the number and arrangement of composite bioelectrodes may depend on the particular application for which a device is used.

Surface potentials measured or recorded by bioelectrodes 210 may be small, and amplification of the biosignals recorded by bioelectrodes 210 may be desired. As shown in FIG. 2, bioelectrodes 210 may be coupled to amplification circuitry (amplifiers) 222 configured to amplify the biosignals conducted by bioelectrodes 210. The output of amplifiers 222 may be provided to analog-to-digital converter (ADC) 224, which may convert the amplified biosignals to digital signals for further processing by a processing device 226. Processing device 226 may be implemented by one or more hardware processors. In some embodiments, amplifiers 222, ADC 224, and/or processing device 226 may represent biosignal-acquiring circuitry that captures a biosignal from a user's body via bioelectrodes 210. The processed signals output from processing device 226 may be interpreted by a host machine 232, examples of which include, but are not limited to, a desktop computer, a laptop computer, a smartwatch, a smartphone, a head-mounted display device, or any other computing device. In some implementations, host machine 232 may be configured to output one or more control signals for controlling a physical or virtual device based, at least in part, on an analysis of the signals output from processing device 226.

As shown in FIG. 2, in addition to bioelectrodes 210, biosignal system 200 may include one or more additional sensors 228, which may be configured to record types of information about a state of a user other than biosignal information. For example, sensors 228 may include temperature sensors for measuring skin/electrode temperature, inertial measurement unit (IMU) sensors for measuring movement information such as rotation and acceleration, humidity sensors for measuring skin/electrode moisture levels, and/or other bio-chemical sensors configured to provide information about the user and/or the user's environment.

According to some examples, sensors 228 may include one or more temperature sensors 229 for measuring temperatures at certain portions of biosignal system 200 and/or regions external to biosignal system 200. In at least one example, biosignal system 200 may include at least one temperature sensor 229 that takes measurements of at least one of bioelectrodes 210. According to some examples, a heating subsystem may be used to raise and/or maintain bioelectrode 210 at a temperature that is above that of a surrounding environment. In some examples, during or following preheating, a bioelectrode temperature may be raised or lowered due to contact with a user's skin and/or due to other temperature changes in the environment surrounding the bioelectrode.

Additionally or alternatively, biosignal system 200 may have a temperature sensor 229 that takes measurements of temperatures from a portion of a user's skin. For example, a temperature sensor 229 may be positioned on a wearable device of biosignal system 200 such that temperature sensor 229 contacts a portion of the user's skin adjacent to the wearable device. In some examples, temperature sensor 229 may be located near at least one of bioelectrodes 210 so that the measured skin temperature is indicative of a temperature in the vicinity of bioelectrodes 210. For example, a user's skin temperature may be elevated or lowered in comparison to surrounding skin regions due to contact with a bioelectrode 210.

Biosignal system 200 may also include a heating subsystem 230. As will be described in greater detail below, heating subsystem 230 may be located in a wearable device and/or may be located external to the wearable device. Heating subsystem 230 may be used to preheat bioelectrodes 210 prior to being worn by a user. Heating subsystem 230 may utilize any type of heating element or other suitable heat source to provide heat sufficient to raise bioelectrodes 210 to a desired temperature. In some examples, heating subsystem 230 may periodically or continually provide heat to raise and/or maintain bioelectrodes 210 at a selected temperature or within a selected temperature range.

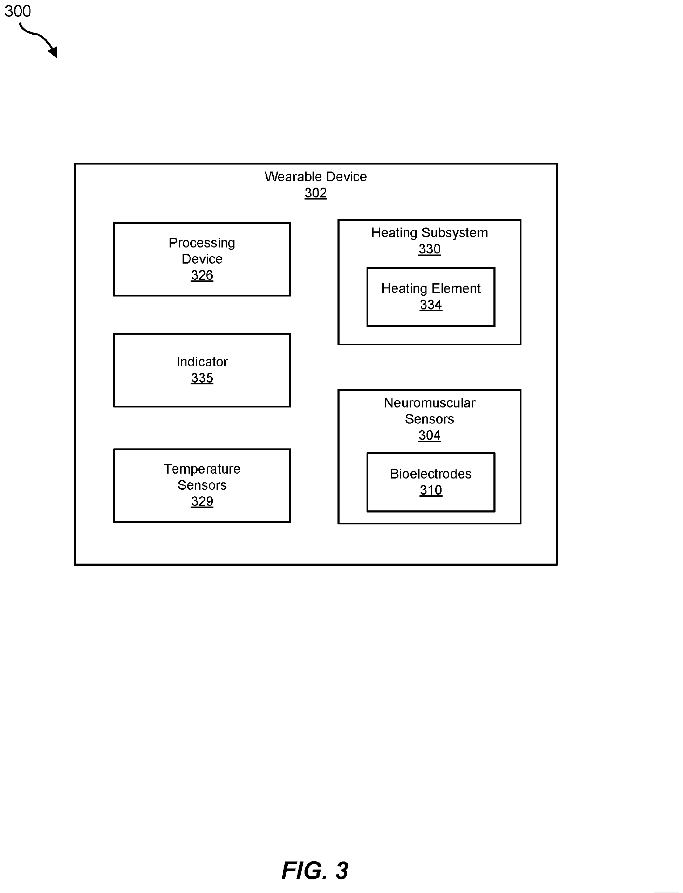

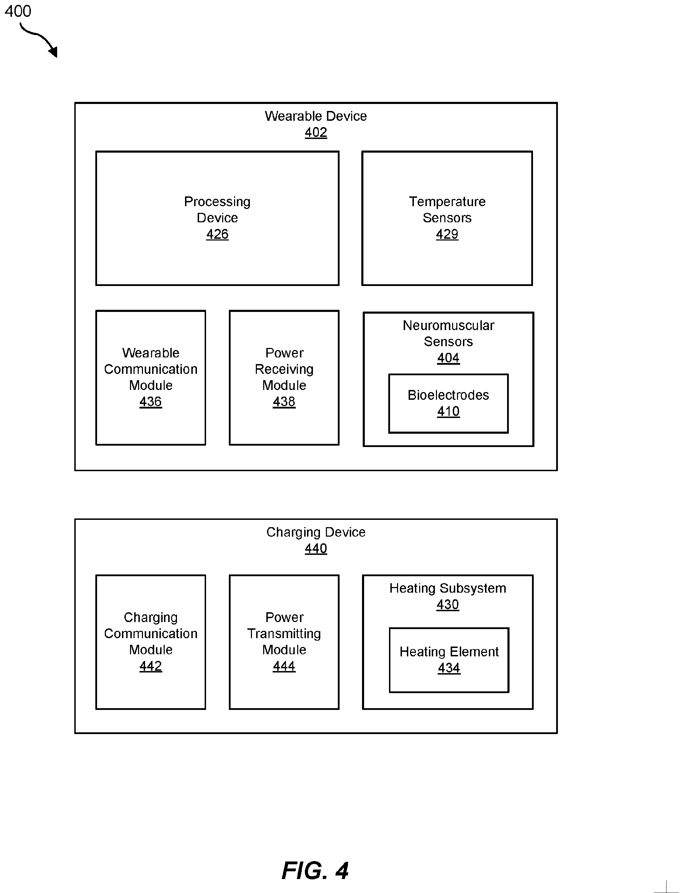

FIGS. 3 and 4 show exemplary embodiments of biosignal systems that include heating subsystems, such as heating subsystem 230 in FIG. 2. For example, FIG. 3 illustrates a biosignal system 300 that includes a wearable device with a built-in heating subsystem and FIG. 4 illustrates a biosignal system 400 that includes a wearable device and a separate device with an external heating subsystem.

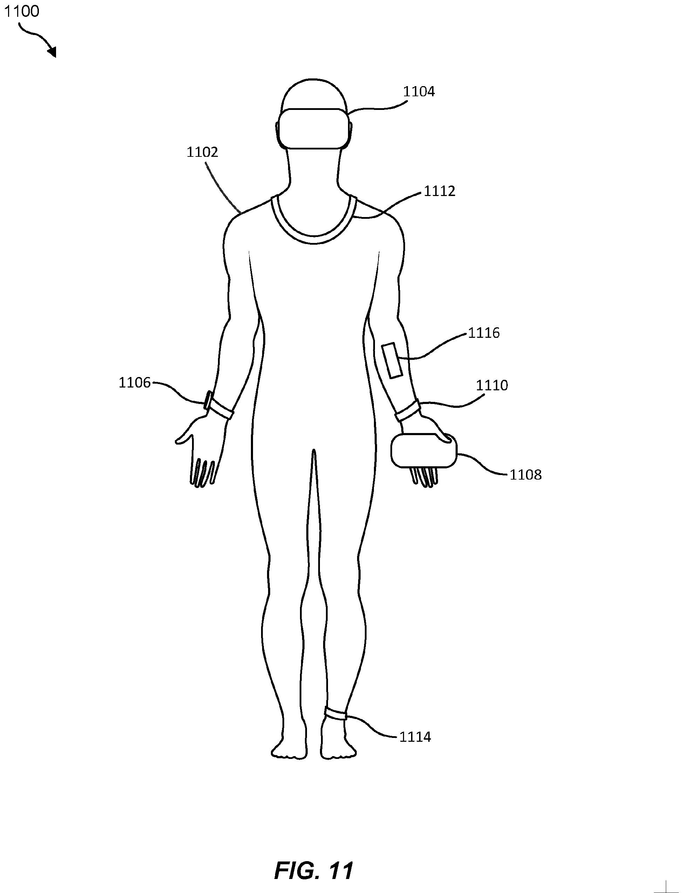

As shown in FIG. 3, exemplary biosignal system 300 includes a wearable device 302. Wearable device 302 may include any suitable type of wearable device having at least a portion that is configured to be worn on at least a portion of a user's body. Wearable device 302 may include, for example, a wrist-worn device, such as wearable device 102 illustrated in FIGS. 1A and 1B and/or a smart watch (see, e.g., FIG. 10). Additionally examples of suitable body-worn wearable devices and systems may include, for example, EMG and ECG wristbands, straps, patches, glasses, etc. Additionally or alternatively, wearable device 302 may include any other suitable device that includes one or more electrodes configured to be worn by a user and/or otherwise contact a user's skin for purposes of measuring biosignals from the user (see, e.g., wearable devices illustrated in FIG. 11).

According to various embodiments, wearable device 302 may include a processing device 326, a temperature sensor 329, and a heating subsystem 330 that includes at least one heating element 334. Wearable device 302 may also include one or more neuromuscular sensors 304, each of which may have at least one bioelectrode 310.

As shown in FIG. 3, heating subsystem 330 may be included in wearable device 302. Heating element 334 of heating subsystem 330 may be disposed in close proximity to one or more of bioelectrodes 310. Heating element 334 may be any suitable type of element capable of transferring heat to bioelectrodes 310. For example, heating element 334 may be a conductive, radiant, resistive, or inductive heating element, and/or any other suitable heating element configured to transmit heat to a surrounding region. Suitable types of heating elements may include, for example, resistance wire, ceramic, semiconductor, thick film, polymer, composite, and/or combination elements.

Temperature sensors 329 may be included in wearable device 302 and may be used to capture temperatures at bioelectrodes 310 and/or at a portion of a user's skin adjacent to bioelectrodes 310. Temperature sensors 329 may include any suitable type of sensor or combination of sensors. For example, temperature sensors 329 may be thermocouples, resistance temperature detectors, thermistors, thermometers, infrared sensors, and/or semiconductor-based integrated circuits. Temperature sensors 329 may be utilized in any suitable manner to determine temperatures prior to and/or during wearable device 302 use. For example, temperature sensors 329 may be used in a feedback loop with heating subsystem 330 to measure bioelectrode/skin temperatures and adjust levels of heating supplied to bioelectrodes 310 by heating element 334.

Wearable device 302 may also include an indicator 335. Indicator 335 may include any suitable notification system for alerting a user to a condition of bioelectrodes 310 and/or heating subsystem 330. Additionally or alternatively, indicator 335 may convey any other suitable information to the user. In some examples, indicator 335 may include a visual notifier, such as a light and/or a display screen, an audible notifier, such as a speaker, and/or a haptic notifier, such as a vibrational motor or other tactile feedback mechanism. In some examples, indicator 335 may provide notifications to a user concerning whether bioelectrodes 310 and/or a portion of the user's skin near bioelectrodes 310 are at or above a threshold temperature and/or within a specified temperature range.

FIG. 4 shows an exemplary biosignal system 400 that includes a portable wearable device 402 that is configured to be worn on at least a portion of a user's body. In the illustrated example, biosignal system 400 also includes a charging device 440 configured to provide power to wearable device 402 for the purpose of recharging batteries of wearable device 402. Additionally, charging device 440 may be configured to heat bioelectrodes of wearable device 402, as described below.

Wearable device 402 may be, for example, a wrist-worn device, such as wearable device 102 illustrated in FIGS. 1A and 1B and/or a smart watch (see, e.g., FIG. 10). Additionally or alternatively, wearable device 402 may include any other suitable device that includes one or more electrodes configured to be worn by a user and/or otherwise contact a user's skin for purposes of measuring biosignals from the user (see, e.g., wearable devices illustrated in FIG. 11). Wearable device 402 may include a processing device 426, a temperature sensor 429, and one or more neuromuscular sensors 404, each of which may have at least one bioelectrode 410.

As additionally shown in FIG. 4, wearable device 402 may include a wearable communication module 436. Wearable communication module 436 may be configured to send and receive communication signals between wearable device 402 and one or more external devices, including charging device 440. In one example, wearable communication module 436 may include one or more antennas and may perform functions related to wireless communications (e.g., WiFi communications, BLUETOOTH communications, cellular communications, etc.) between wearable device 402 and at least one external computing device (e.g., a gaming and/or multimedia console or device, a desktop, a laptop, a tablet, a cellular phone, a smart phone, a wearable device, an embedded system, an internet router, another head-mounted-display device, a hand-held controller, etc.).

Wearable communication module 436 may communicate via a wireless and/or wired connection with external devices either directly or via a suitable network, such as, for example, an intranet, a Wide Area Network (WAN), a Local Area Network (LAN), a low power wide area network (LPWAN), a Personal Area Network (PAN), the Internet, Power Line Communications (PLC), a cellular network (e.g., a Global System for Mobile Communications (GSM) network), portions of one or more of the same, variations or combinations of one or more of the same, and/or any other suitable network. Examples of wearable communication module 436 include, without limitation, WiFi and/or BLUETOOTH chipsets, WiFi and/or BLUETOOTH microcontrollers, and/or any other suitable communication device.

Wearable device 402 also includes a power receiving module 438 for receiving power from one or more external devices, such as from charging device 440. In some examples, power receiving module 438 may be part of or may include a power management device. Power receiving module 438 may include, for example, any type or form of physical device for performing power control functions, such as converting electronic power (e.g., battery power) for use by various components of wearable device 402. In some examples, power receiving module 438 may perform functions related to direct current (DC) to DC power conversion, power-source selection, battery and/or capacitor charging, power sequencing, pulse-frequency and/or pulse-width modulation, and/or voltage scanning. Examples of power receiving module 438 include, without limitation, power management integrated circuits (e.g., PMICs, PMUs, etc.), power management chips, power management system blocks, and/or any other suitable power management device.

Power receiving module 438 may receive power from charging device 440 in any suitable manner. For example, power receiving module 438 may receive power via a wired connection (e.g., via a charging cord connected to a port of wearable device 402). Additionally or alternatively, power receiving module 438 may receive power wirelessly via, for example, inductive charging. For example, power receiving module 438 may include a secondary inductive coil that receives power induced by a primary coil of a wireless charging device, such as charging device 440.

As illustrated in FIG. 4, charging device 440 may include a heating subsystem 430 having at least one heating element 434 configured to heat bioelectrodes 410 when wearable device 402 is disposed in close proximity to charging device 440. According to some embodiments, charging device 440 may function as a charging dock or station that is dimensioned to hold and/or otherwise abut wearable device 402 for purposes of charging and/or heating. In some examples, charging device 440 may also perform additional functions. For example, charging device 440 may function as a physical storage member for securely holding wearable device 402. Additionally or alternatively, charging device 440 may transfer data to or from wearable device 402.

Charging device 440 may also include a charging communication module 442 and a power transmitting module 444. Charging communication module 442 may be configured to communicate with wearable device 402 and/or any other suitable external device. In one example, charging communication module 442 may include one or more antennas and may perform functions related to wireless communications (e.g., WiFi communications, BLUETOOTH communications, cellular communications, etc.) between charging device 440 and wearable device 402 and/or at least one external computing device (e.g., a gaming and/or multimedia console or device, a desktop, a laptop, a tablet, a cellular phone, a smart phone, a wearable device, an embedded system, an internet router, another head-mounted-display device, a hand-held controller, etc.). Charging communication module 442 may communicate via a wireless and/or wired connection with external devices either directly or via a suitable network, such as, for example, an intranet, a WAN, a LAN, a LPWAN, a PAN, the Internet, PLC, a cellular network, portions of one or more of the same, variations or combinations of one or more of the same, and/or any other suitable network. Examples of charging communication module 442 include, without limitation, WiFi and/or BLUETOOTH chipsets, WiFi and/or BLUETOOTH microcontrollers, and/or any other suitable communication device.

Charging device 440 also includes a power transmitting module 444 for transmitting power to one or more external devices, including wearable device 402. Power transmitting module 444 may transmit power to power receiving module 438 of wearable device 402 in any suitable manner. For example, power transmitting module 444 may transmit power to power receiving module 438 via a wired connection (e.g., via a charging cord and/or connector coupled to a port of wearable device 402). Additionally or alternatively, power transmitting module 444 may transmit power to power receiving module 438 wirelessly via, for example, inductive charging. For example, power transmitting module 444 may include a primary coil that induces a current in a secondary coil of power receiving module 438 when wearable device 402 is disposed on or in close proximity to charging device 440.

As shown in FIG. 4, at least one heating element 434 of heating subsystem 430 may be disposed in close proximity to one or more of bioelectrodes 410. Heating element 434 may be any suitable type of element capable of transferring heat to bioelectrodes 410. For example, heating element 434 may be a conductive, radiant, resistive, or inductive heating element, and/or any other suitable heating element configured to transmit heat to a surrounding region. Suitable types of heating elements may include, for example, resistance wire, ceramic, semiconductor, thick film, polymer, composite, and/or combination elements.

Temperature sensors 429 may be included in wearable device 402 and may be used to capture temperatures at bioelectrodes 410 and/or at a portion of a user's skin adjacent to bioelectrodes 410. Additionally or alternatively, one or more temperature sensors may be included in charging device 440. Temperature sensors 429 may include any suitable type of sensor or combination of sensors. For example, temperature sensors 429 may be thermocouples, resistance temperature detectors, thermistors, thermometers, infrared sensors, and/or semiconductor-based integrated circuits. Temperature sensors 429 may be utilized in any suitable manner to determine temperatures prior to and/or during wearable device 402 use. For example, temperature sensors 429 may be used in a feedback loop with heating subsystem 430 to measure temperatures of bioelectrode 410 and adjust levels of heating supplied to bioelectrodes 410 by heating element 434.

In biosignal system 400 illustrated in FIG. 4, bioelectrodes 410 in wearable device 402 may be preheated by heating elements of an external device prior to wearable device 402 being worn by a user. In some examples, charging device 440 may detect that wearable device 402 is coupled to charging device 440, either by a cable/connector or wirelessly. Heating subsystem 430 may then turn on heating element 434 so that one or more of bioelectrodes 410 of neuromuscular sensors 404 are raised and/or maintained at a desired temperature and/or within a desired temperature range. In some examples, at least one of bioelectrodes 410 may be raised to a temperature at or above a threshold temperature. The threshold temperature may, for example, be a temperature at which performance of bioelectrodes 410 is substantially optimized. The temperature(s) of bioelectrodes 410 may be determined, for example, by one or more of temperature sensors 429. Additionally or alternatively, charging device 440 may include one or more temperature sensors for measuring temperatures of bioelectrodes 410.

Wearable device 402 and/or charging device 440 may also include an indicator (see, e.g., indicator 335 in FIG. 3). The indicator may provide notifications to a user concerning whether bioelectrodes 410 and/or a portion of the user's skin near bioelectrodes 410 are at or above a threshold temperature and/or within a specified temperature range. In some examples, in addition to heating subsystem 430, wearable device 402 may also include a separate heating subsystem with its own heating element(s) (see, e.g., heating subsystem 330 in FIG. 3). Such an internal heating subsystem may be utilized when, for example, wearable device 402 is not docked to and/or disposed in close proximity to charging device 440 (e.g., when wearable device 402 is worn by a user, when wearable device 402 is coupled to another charging device that does not include a heating subsystem, etc.).

Increasing the surface temperature of dry bioelectrodes (e.g., dry metal, polymer, composite electrodes, etc.) to slightly above a user's skin temperature may have a positive impact on lowering initial skin-electrode impedance. For example, when the surface temperature of the dry bioelectrodes is increased from around a standard room temperature (e.g., approximately 23 °C) to approximately 35 °C (within the bounds of physiological human skin temperature and comfort), an approximately 46% drop in initial impedance may be observed. The improvement at the skin-electrode interface may be attributed, for example, to heat-induced activation of regional sweat glands, moisture build-up at the interface, and/or hydration of the stratum corneum layer. Bioelectrodes may be raised to any suitable temperature, such as a temperature at or above a threshold temperature of approximately 30 °C or more. In some examples, bioelectrodes may be raised to a temperature at or above a threshold temperature within a range of from approximately 30 °C to approximate 40 °C (e.g., approximately 30 °C, approximately 31 °C, approximately 32 °C, approximately 33 °C, approximately 34 °C, approximately 35 °C, approximately 36 °C, approximately 37 °C, approximately 38 °C, approximately 39 °C, approximately 40 °C).

Preheating bioelectrodes prior to wear may result in low initial impedance, which may in turn provide high accessibility to important information when the device is newly worn by a user. In contrast, bioelectrodes that are not preheated may return inaccurate and/or inconclusive data results during an initial period prior to warming by the user's own body heat. According to various embodiments, when the temperature of a bioelectrode is at or above a threshold temperature of approximately 30 °C or more, skin-electrode impedance values may be substantially optimized and/or improved in comparison to bioelectrodes at temperatures below 30 °C. For example, initial skin-electrode impedance values for bioelectrodes having initial temperatures at or above a threshold temperature of 30 °C or more may be 0.6 MΩ or less (e.g., approximately 0.60 MΩ, approximately 0.55 MΩ, approximately 0.50 MΩ, approximately 0.45 MΩ, approximately 0.40 MΩ, approximately 0.35 MΩ, approximately 0.30 MΩ, approximately 0.25 MΩ, approximately 0.20 MΩ, approximately 0.15 MΩ, approximately 0.10 MΩ, approximately 0.05 MΩ).

According to some examples, electrodes may be maintained at a desired temperature while being worn by a user. For example, one or more bioelectrodes may be maintained at a temperature that is above a user's skin temperature while a wearable device including the bioelectrodes is worn. Temperatures above the user's skin temperature may result in further reductions in skin-electrode impedance in comparison to electrodes maintained at or near the skin temperature. Accordingly, low continued skin-electrode impedance and fast motion artifact recovery may be realized while the bioelectrodes are kept warm during use.

In addition to improvements in bioelectrode functionality and reduced skin-electrode impedance, heating/preheating of the electrodes may provide other advantages. For example, preheating bioelectrodes of a wearable device prior to use and/or heating the bioelectrodes during use may improve user perception and comfort. For example, the bioelectrodes may be heated to a temperature that is within a physiological skin temperature range (e.g., approximately 33 °C - 37 °C)

Heating and/or preheating can be applied to body-worn wearable devices and integrated with many wearable technologies, such as EMG and ECG wristbands, straps, patches, glasses, etc., in a variety of ways. For example, the temperatures of recording bioelectrodes may be adjusted to preset values during wireless charging, which may ensure that the surface temperatures of the dry electrodes are held at fixed values before the wearable device is worn. Preheating bioelectrodes prior to wear may be battery-friendly since energy from wireless charging is used rather than draining a wireless device battery. The heat may be supplied to the bioelectrodes either from a heating element within the wireless device itself (see, e.g., heating element 334 in FIG. 3) during charging, from a heating element within the charging device (see, e.g., heating element 434 in FIG. 4), and/or from any other suitable external heat source.

According to at least one example, temperatures of bioelectrodes may be adjusted based on measured user skin temperatures via a feedback system. This approach may utilize external temperature sensors for measuring skin temperatures. A conductive and/or radiant heating element or thermoelectric device may be used to increase or decrease electrode temperatures. In some examples, a controller may be utilized for processing temperature data and maintaining electrodes at set temperatures. Such a controller may be located, for example, within a wearable device and/or a host machine communicatively couple to the wearable device.

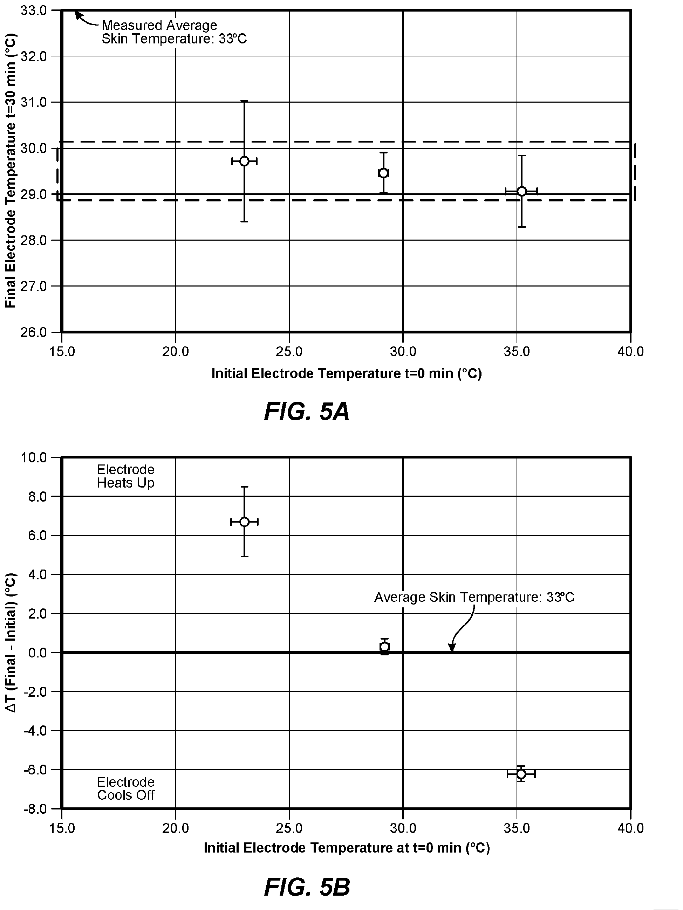

In accordance with some examples, effects of electrode surface temperatures on skin-electrode impedance parameters were evaluated. FIGS. 5A and 5B illustrate relationships between initial and final electrode temperatures after being worn by a user for a period of 30 minutes, in accordance with various embodiments.

In some examples, three gold plated electrodes (i.e., bioelectrodes) having diameters of 9 mm were heated on a temperature controlled hot plate to increase their temperatures. Surface temperatures of the electrodes were measured with a K type thermocouple. Additionally, surface temperatures of a test site on a user's skin were measured with a K type thermocouple. Skin-electrode impedance measurements were carried out with a PALMSENS4 Impedance Analyzer at 100 Hz for 30 min. Statistical analysis was performed using t-test comparison (two-sample assuming unequal variances).

As shown in FIGS. 5A and 5B, initial surface temperatures of each of the three electrodes were measured to be approximately 23 °C (i.e., room temperature), approximately 29 °C, and approximately 35 °C. The measured average skin temperature was measured to be approximately 33 °C. During heating with a heating element, the surface temperatures of the metal electrodes increased and approached the local skin temperature of the user. At 30 min, temperature differences between the electrode surfaces and the user's skin were approximately 3-4 °C. For example, as shown in FIG. 5A, final electrode temperatures after being in contact with the user's skin for approximately 30 minutes were in the range of approximately 29-30 °C. FIG. 5B illustrates changes in temperature (ΔT) between the final and initial electrode temperatures, with electrodes either heating, cooling, or remaining substantially constant based on whether the initial electrode temperatures were higher or lower than the user's skin temperature.

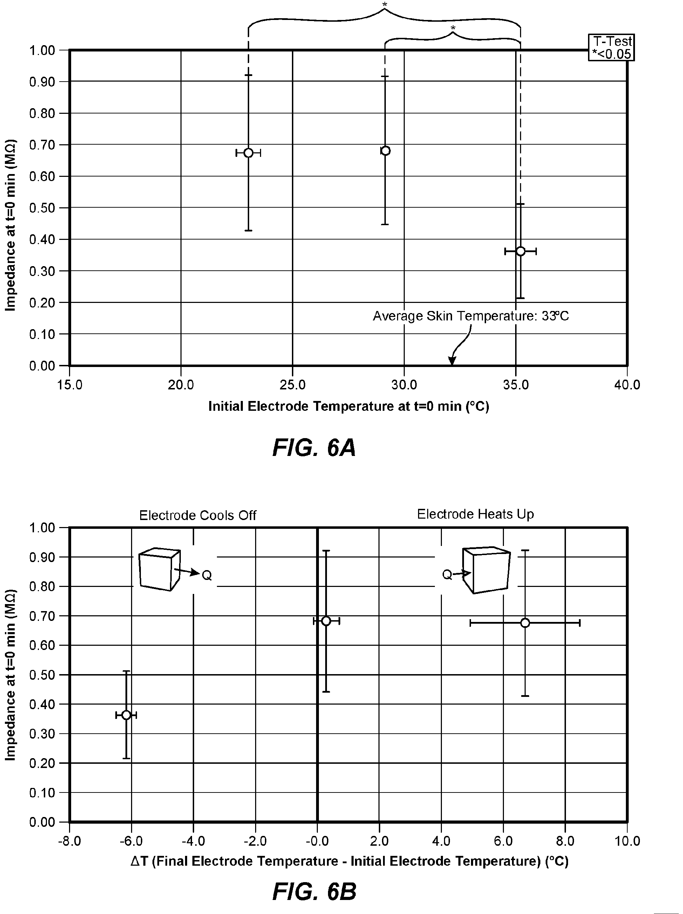

FIGS. 6A and 6B illustrate relationships between initial impedance values and electrode temperatures, in accordance with various embodiments. If an initial surface temperature of the metal electrode was higher than the user's skin temperature, the initial starting impedance was demonstrated to be approximately 46% lower than cases where the initial surface temperature was below the skin temperature. For example, as shown in FIG. 6A, when the initial temperature of the electrode was 35 °C (i.e., above the initial skin temperature of 33 °C), the skin-electrode impedance was 0.36 MΩ. In contrast, when the initial temperature of the electrode was 23 °C (i.e., below the initial skin temperature), the skin-electrode impedance was 0.67 MΩ, and when the initial temperature of the electrode was 29 °C (i.e., still below the initial skin temperature), the skin-electrode impedance was 0.68 MΩ. FIG. 6B illustrates that the initial impedance value (i.e., 0.36 MΩ) for the electrode that cooled from a preheated temperature during wear was significantly lower than the initial impedance values (0.67 MΩ and 0.68 MΩ) for the electrodes that were warmed by the user's skin or remained approximately constant during wear.

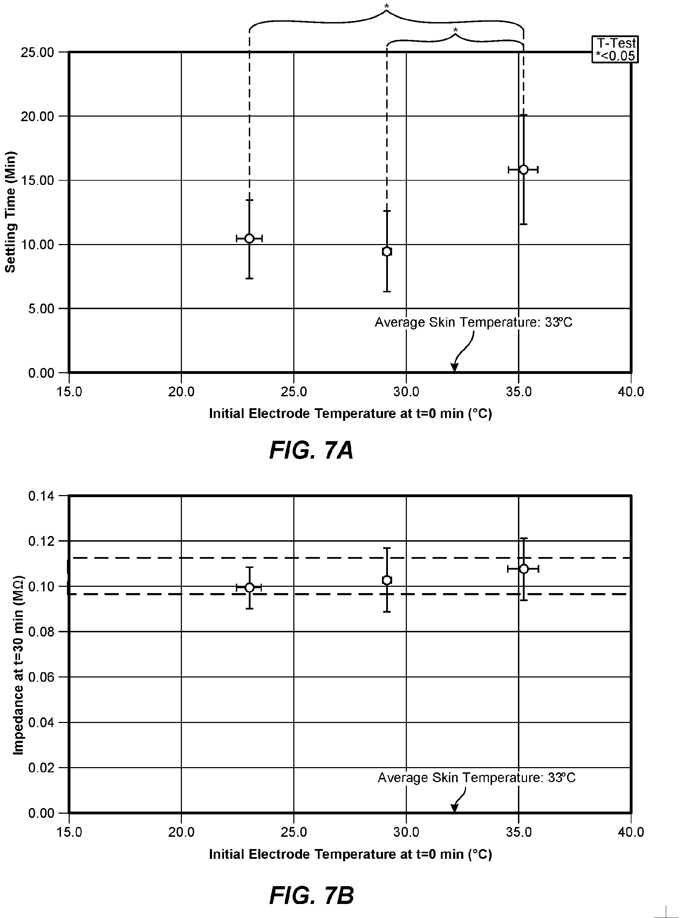

FIGS. 7A and 7B show settling times and impedance values over time, in accordance with various embodiments. As illustrated in FIG. 7A, settling times went up as the initial electrode surface temperature was increased from room temperature (approximately 23 °C) to approximately 35 °C. However, as indicated in FIG. 7B, the initial temperature did not affect final impedance values at a time of 30 min, with the final impedance values being statistically insignificant.

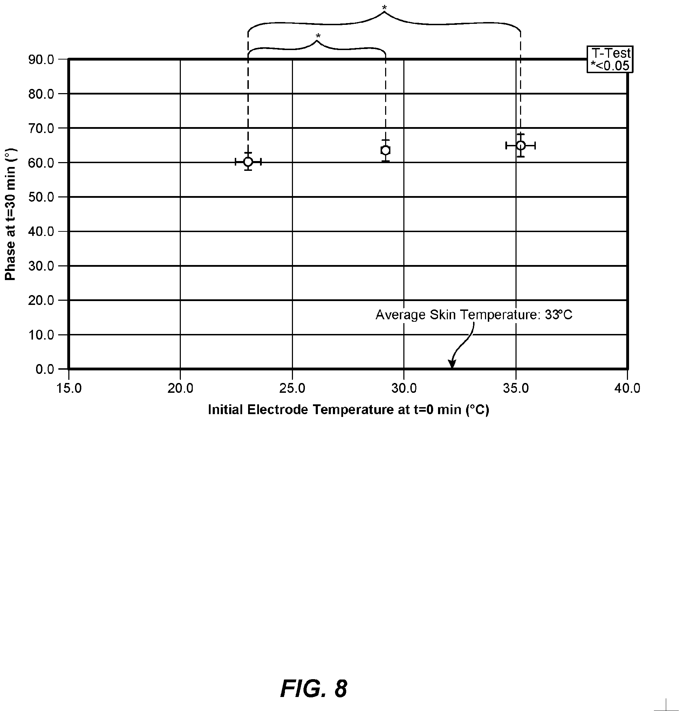

FIG. 8 shows phase values versus initial electrode temperature, in accordance with some embodiments. As shown, phase values increased moderately as the initial electrode surface temperature was increased from approximately 23 °C to approximately 35 °C. The difference between mean final phase values at 30 minutes for each of the electrodes was determined to be statistically significant.

FIG. 9 is a flow diagram of an exemplary method 900 for operating a biopotential measurement system having an electrode preheating unit, according to some embodiments. As illustrated in FIG. 9, at step 910, at least one bioelectrode of a wearable device may be heated such that a measured bioelectrode temperature of the at least one bioelectrode is at or above a threshold temperature.

At step 920 in FIG. 9, the at least one bioelectrode may be positioned abutting a user's skin when the wearable device is worn by the user. For example, bioelectrodes 110 of wearable system 100 shown in FIGS. 1A and 1B may be disposed on an inner-facing portion of wearable device 102 so that electrode surfaces 116 of bioelectrodes 110 are positioned abutting a user's skin when wearable device 102 is worn. Additional wearable devices shown and described herein may include bioelectrodes that are configured to abut a user's skin during use (see, e.g., FIGS. 10-17B).

At step 930 in FIG. 9, a biosignal may be captured from a user's body via the at least one bioelectrode. For example, amplifiers 222, ADC 224, and/or processing device 226 may represent biosignal-acquiring circuitry that captures a biosignal from a user's body via bioelectrodes 210 (see, e.g., FIG. 2).

In some examples, an additional temperature of at least one of the at least one bioelectrode and a portion of the user's skin may be measured. In this example, a level of heat applied to the at least one bioelectrode may be adjusted based on the measured additional temperature.

As described herein, the disclosed biopotential measurement systems and devices may include heating subsystems that preheat electrodes prior to being worn by a user. Preheating the electrodes to a specified temperature or range, such as a temperature higher than the user's skin temperature, may significantly improve electrode performance during an initial period after a user begins wearing a device. Such improvements may enable the capture of more accurate biosignal data within a shortened window of time. Initial impedance values between an electrode and a user's skin may be reduced by such preheating, resulting in greatly reduced signal-to-noise ratios and improved signal quality. Additionally, preheating the electrodes may warm the electrodes to a temperature closer to the user's skin temperature, thereby improving user comfort when the device is worn. The disclosed systems and methods may also eliminate the need to use invasive or uncomfortable skin preparations in combination with dry electrodes.

### Example Embodiments

Example 1: A biopotential measurement system including a wearable device that includes 1) at least one bioelectrode positioned to abut a user's skin when the wearable device is worn by the user and 2) biosignal-acquiring circuitry that captures a biosignal from the user's body via the at least one bioelectrode, where measurement performance of the at least one bioelectrode is substantially optimized when a temperature of the at least one bioelectrode is at or above a threshold temperature.

Example 2: The biopotential measurement system of Example 1, further including a temperature sensor for measuring a temperature of at least one of the at least one bioelectrode or a portion of the user's skin.

Example 3: The biopotential measurement system of any of Examples 1 and 2, where the threshold temperature includes a temperature of approximately 30 °C or more.

Example 4: The biopotential measurement system of any of Examples 1-3, where a skin-electrode impedance of the at least one bioelectrode is approximately 0.6 MΩ or less when the temperature of the at least one bioelectrode is at or above the threshold temperature.

Example 5: The biopotential measurement system of any of Examples 1-4, where the wearable device is configured to extend around at least a portion of the user's arm or head and the at least one bioelectrode is positioned at an inner surface portion of the wearable device.

Example 6: The biopotential measurement system of any of Examples 1-5, further including an indicator that provides notification to the user of whether the bioelectrode is at or above the threshold temperature.

Example 7: The biopotential measurement system of any of Examples 1-6, where the at least one bioelectrode includes a metal material.

Example 8: The biopotential measurement system of any of Examples 1-7, where the biopotential measurement system further includes a heating subsystem that heats the at least one bioelectrode.

Example 9: The biopotential measurement system of Example 8, where the heating subsystem preheats the at least one bioelectrode to the temperature at or above the threshold temperature when the wearable device is not worn by the user.

Example 10: The biopotential measurement system of any of Examples 8 and 9, where the heating subsystem is located in the wearable device.

Example 11: The biopotential measurement system of any of Examples 8-10, further including a charging device for providing power to the wearable device, where the heating subsystem is located in the charging device.

Example 12: The biopotential measurement system of Example 11, where the charging device includes a charging dock and the heating subsystem heats the at least one bioelectrode when the wearable device is disposed on the charging dock.

Example 13: The biopotential measurement system of any of Examples 8-12, where heating subsystem includes a heating element.

Example 14: The biopotential measurement system of Example 13, where the heating element is disposed in close proximity to the bioelectrode.

Example 15: The biopotential measurement system of any of Examples 13 and 14, where the heating element includes at least one of a conductive heating element or a radiant heating element.

Example 16: The biopotential measurement system of any of Examples 1-15, where the at least one bioelectrode includes a plurality of electrodes disposed at different locations.

Example 17: A wearable device including 1) at least one bioelectrode positioned to abut a user's skin when the wearable device is worn by the user, 2) biosignal-acquiring circuitry that captures a biosignal from the user's body via the at least one bioelectrode, and 3) a heating subsystem that heats the at least one bioelectrode.

Example 18: A method including 1) heating at least one bioelectrode of a wearable device such that a temperature of the at least one bioelectrode is at or above a threshold temperature, 2) positioning the at least one bioelectrode abutting a user's skin when the wearable device is worn by the user, and 3) capturing a biosignal from the user's body via the at least one bioelectrode.

Example 19: The method of Example 18, where the threshold temperature includes a temperature of approximately 30 °C or more.

Example 20: The method of Example 19, further including heating the at least one bioelectrode prior to positioning the at least one bioelectrode abutting the user's skin.

The bioelectrodes and biosignal systems disclosed herein may be implemented into, conformed to, and/or suitably shaped to fit a variety of wearable devices. In some examples, the terms "wearable" and "wearable device" may refer to any type or form of computing device that is worn by a user of an artificial-reality system and/or visual display system as part of an article of clothing, an accessory, and/or an implant. In one example, a wearable device may include and/or represent a wristband secured to and/or worn by the wrist of a user. Additional examples of wearable devices include, without limitation, armbands, pendants, bracelets, rings, jewelry, anklebands, clothing, electronic textiles, shoes, clips, headsets, headbands, head-mounted displays, gloves, glasses, variations or combinations of one or more of the same, and/or any other suitable wearable devices.

Embodiments of the present disclosure may include or be implemented in conjunction with various types of wearable devices. FIG. 10 illustrates an example system 1000 that includes a watch body 1004 coupled to a wristband 1012. Watch body 1004 and wristband 1012 may have any size and/or shape that is configured to allow a user to wear system 1000 on a body part (e.g., a wrist). System 1000 may perform various functions associated with the user. The functions may be executed independently in watch body 1004, independently in wristband 1012, and/or in communication between watch body 1004 and wristband 1012. Functions executed by system 1000 may include, without limitation, display of visual content to the user (e.g., visual content displayed on display screen 1002), sensing user input (e.g., sensing a touch on button 1008, sensing biometric data or neuromuscular signals with composite bioelectrodes 210, messaging (e.g., text, speech, video, etc.), image capture, wireless communications (e.g., cellular, near field, WiFi, personal area network, etc.), location determination, financial transactions, providing haptic feedback, etc. Functions may be executed on system 1000 in conjunction with an artificial-reality system.

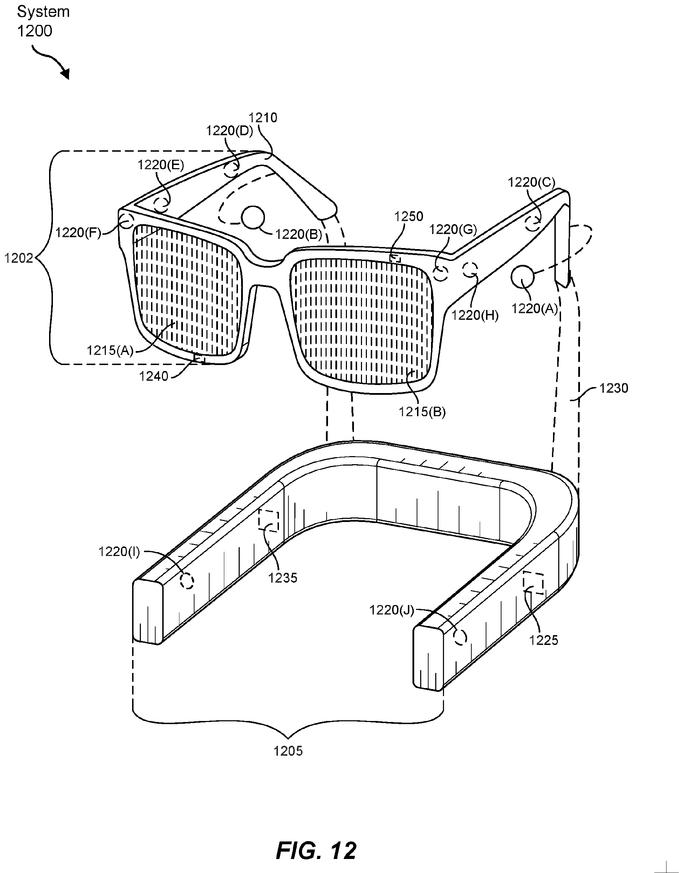

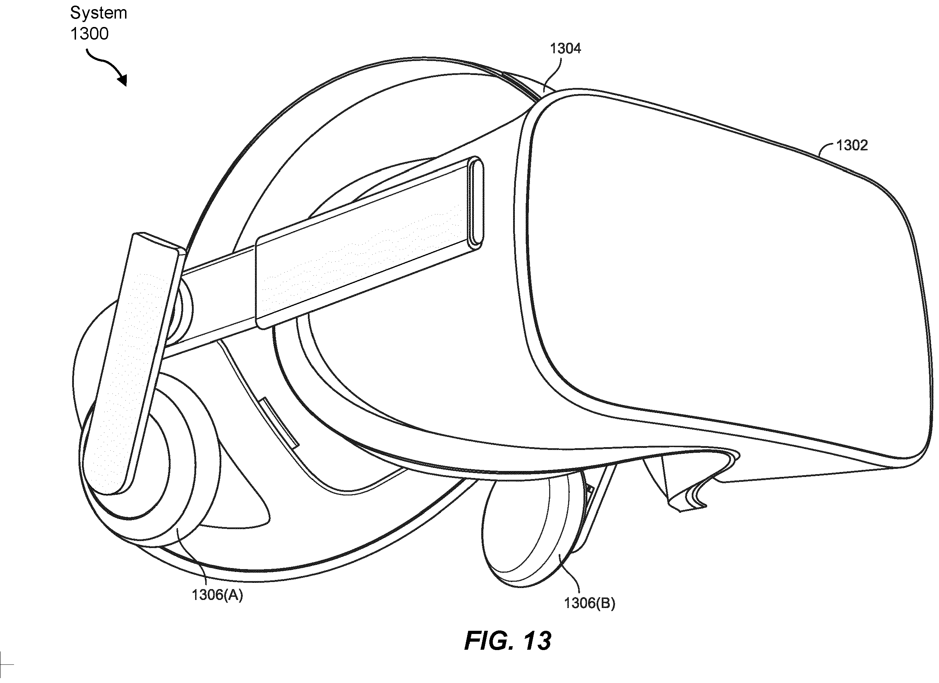

Wristband 1012 may be donned (e.g., worn) on a body part (e.g., a wrist) of a user and may operate independently from watch body 1004. For example, wristband 1012 may be configured to be worn by a user and an inner surface of wristband 1012 may be in contact with the user's skin. When worn by a user, composite bioelectrodes 210 may be in contact with the user's skin. An electromyography sensor may, for example, be integrated into wristband 1012 and may sense a user's muscle intention. The sensed muscle intention may be transmitted to an artificial reality system (e.g., the augmented-reality system 1200 in FIG. 12 or the virtual-reality system 1300 in FIG. 13) to perform an action in an associated artificial-reality environment, such as to control a physical and/or virtual object displayed to the user.

The disclosed biosignal systems may be implemented into one or more of the devices in example systems 1100 shown in FIG. 11. As illustrated in this figure, system 1100 may include a user 1102 and computing devices that are worn or held by user 1102. For example, FIG. 11 shows a head-mounted display system 1104, such as head-mounted display device 1200 illustrated in FIG. 12 or head-mounted display system 1300 shown in FIG. 13, worn on the head of user 1102. FIG. 11 further shows a smart watch 1106 worn on a wrist of user 1102, a smart phone 1108 or other portable device held in a hand of user 1102, an electronic device 1110 worn on a wrist of user 1102, an electronic device 1112 worn about the neck region of user 1102, an electronic device 1114 worn on an ankle of user 1102, and a flexible electronic device 1116 worn on a forearm of user 1102. In some examples, one or more of the devices shown in FIG. 11 may be shaped to conform to a corresponding portion of the wearer's body.

The various devices, systems, and methods described herein may involve the use of a wearable device capable of detecting and/or sensing neuromuscular signals traversing through a user's body. For example, a user may wear a smart wristband with multiple surface electromyography (EMG) sensors that detect and/or sense neuromuscular signals traversing the user's arm, wrist, and/or hand. In this example, the smart wristband may be communicatively coupled to a nearby computing device. In response to certain neuromuscular signals detected via the user's body, the smart wristband may direct the computing device to perform one or more actions that account for those neuromuscular signals.

Accordingly, the smart wristband may enable the user to engage with interactive media presented and/or displayed on the computing device in less restrictive ways than traditional HCls. The smart wristband may be used to control certain elements of interactive media based at least in part on EMG signals that correlate to predefined states of one or more body parts of the user. The smart wristband may enable the user to direct the computing device to perform certain interactive tasks. Examples of such interactive tasks include, without limitation, map navigation, page browsing, gaming controls, flight controls, interactions with graphical objects presented on a display, cursor control, link and/or button selection, combinations of one or more of the same, and/or any other suitable interactive tasks.

In some implementations, a wearable device may be used to transition between different mappings of body part states and responsive actions. For example, the wearable device may detect and/or sense certain neuromuscular signals traversing a user's body. In this example, those neuromuscular signals may correspond to and/or represent a specific state of one or more of the user's body parts. As a result, the wearable device may be able to detect and/or sense one or more positions, movements, forces, contractions, poses, and/or gestures made by those body parts of the user. One mapping may cause the wearable device and/or the target computing device to perform a certain action in response to the detection of a specific state of those body parts. However, another mapping may cause the wearable device and/or the target computing device to perform a different action in response to the detection of the same state of those body parts. The wearable device may enable the user to transition between those mappings via neuromuscular signals.

Embodiments of the present disclosure may include or be implemented in conjunction with various types of artificial-reality systems. Artificial reality is a form of reality that has been adjusted in some manner before presentation to a user, which may include, for example, a virtual reality, an augmented reality, a mixed reality, a hybrid reality, or some combination and/or derivative thereof. Artificial-reality content may include completely computer-generated content or computer-generated content combined with captured (e.g., real-world) content. The artificial-reality content may include video, audio, haptic feedback, or some combination thereof, any of which may be presented in a single channel or in multiple channels (such as stereo video that produces a three-dimensional (3D) effect to the viewer). Additionally, in some embodiments, artificial reality may also be associated with applications, products, accessories, services, or some combination thereof, that are used to, for example, create content in an artificial reality and/or are otherwise used in (e.g., to perform activities in) an artificial reality.

Artificial-reality systems may be implemented in a variety of different form factors and configurations. Some artificial-reality systems may be designed to work without near-eye displays (NEDs). Other artificial-reality systems may include an NED that also provides visibility into the real world (such as, e.g., augmented-reality system 1200 in FIG. 12) or that visually immerses a user in an artificial reality (such as, e.g., virtual-reality system 1300 in FIG. 13). While some artificial-reality devices may be self-contained systems, other artificial-reality devices may communicate and/or coordinate with external devices to provide an artificial-reality experience to a user. Examples of such external devices include handheld controllers, mobile devices, desktop computers, devices worn by a user, devices worn by one or more other users, and/or any other suitable external system.

Turning to FIG. 12, augmented-reality system 1200 may include an eyewear device 1202 with a frame 1210 configured to hold a left display device 1215(A) and a right display device 1215(B) in front of a user's eyes. Display devices 1215(A) and 1215(B) may act together or independently to present an image or series of images to a user. While augmented-reality system 1200 includes two displays, embodiments of this disclosure may be implemented in augmented-reality systems with a single NED or more than two NEDs.

In some embodiments, augmented-reality system 1200 may include one or more sensors, such as sensor 1240. Sensor 1240 may generate measurement signals in response to motion of augmented-reality system 1200 and may be located on substantially any portion of frame 1210. Sensor 1240 may represent one or more of a variety of different sensing mechanisms, such as a position sensor, an inertial measurement unit (IMU), a depth camera assembly, a structured light emitter and/or detector, or any combination thereof. In some embodiments, augmented-reality system 1200 may or may not include sensor 1240 or may include more than one sensor. In embodiments in which sensor 1240 includes an IMU, the IMU may generate calibration data based on measurement signals from sensor 1240. Examples of sensor 1240 may include, without limitation, accelerometers, gyroscopes, magnetometers, other suitable types of sensors that detect motion, sensors used for error correction of the IMU, or some combination thereof.

In some examples, augmented-reality system 1200 may also include a microphone array with a plurality of acoustic transducers 1220(A)-1220(J), referred to collectively as acoustic transducers 1220. Acoustic transducers 1220 may represent transducers that detect air pressure variations induced by sound waves. Each acoustic transducer 1220 may be configured to detect sound and convert the detected sound into an electronic format (e.g., an analog or digital format). The microphone array in FIG. 12 may include, for example, ten acoustic transducers: 1220(A) and 1220(B), which may be designed to be placed inside a corresponding ear of the user, acoustic transducers 1220(C), 1220(D), 1220(E), 1220(F), 1220(G), and 1220(H), which may be positioned at various locations on frame 1210, and/or acoustic transducers 1220(1) and 1220(J), which may be positioned on a corresponding neckband 1205.

In some embodiments, one or more of acoustic transducers 1220(A)-(J) may be used as output transducers (e.g., speakers). For example, acoustic transducers 1220(A) and/or 1220(B) may be earbuds or any other suitable type of headphone or speaker.

The configuration of acoustic transducers 1220 of the microphone array may vary. While augmented-reality system 1200 is shown in FIG. 12 as having ten acoustic transducers 1220, the number of acoustic transducers 1220 may be greater or less than ten. In some embodiments, using higher numbers of acoustic transducers 1220 may increase the amount of audio information collected and/or the sensitivity and accuracy of the audio information. In contrast, using a lower number of acoustic transducers 1220 may decrease the computing power required by an associated controller 1250 to process the collected audio information. In addition, the position of each acoustic transducer 1220 of the microphone array may vary. For example, the position of an acoustic transducer 1220 may include a defined position on the user, a defined coordinate on frame 1210, an orientation associated with each acoustic transducer 1220, or some combination thereof.

Acoustic transducers 1220(A) and 1220(B) may be positioned on different parts of the user's ear, such as behind the pinna, behind the tragus, and/or within the auricle or fossa. Or, there may be additional acoustic transducers 1220 on or surrounding the ear in addition to acoustic transducers 1220 inside the ear canal. Having an acoustic transducer 1220 positioned next to an ear canal of a user may enable the microphone array to collect information on how sounds arrive at the ear canal. By positioning at least two of acoustic transducers 1220 on either side of a user's head (e.g., as binaural microphones), augmented-reality device 1200 may simulate binaural hearing and capture a 3D stereo sound field around about a user's head. In some embodiments, acoustic transducers 1220(A) and 1220(B) may be connected to augmented-reality system 1200 via a wired connection 1230, and in other embodiments acoustic transducers 1220(A) and 1220(B) may be connected to augmented-reality system 1200 via a wireless connection (e.g., a BLUETOOTH connection). In still other embodiments, acoustic transducers 1220(A) and 1220(B) may not be used at all in conjunction with augmented-reality system 1200.

Acoustic transducers 1220 on frame 1210 may be positioned in a variety of different ways, including along the length of the temples, across the bridge, above or below display devices 1215(A) and 1215(B), or some combination thereof. Acoustic transducers 1220 may also be oriented such that the microphone array is able to detect sounds in a wide range of directions surrounding the user wearing the augmented-reality system 1200. In some embodiments, an optimization process may be performed during manufacturing of augmented-reality system 1200 to determine relative positioning of each acoustic transducer 1220 in the microphone array.

In some examples, augmented-reality system 1200 may include or be connected to an external device (e.g., a paired device), such as neckband 1205. Neckband 1205 generally represents any type or form of paired device. Thus, the following discussion of neckband 1205 may also apply to various other paired devices, such as charging cases, smart watches, smart phones, wrist bands, other wearable devices, hand-held controllers, tablet computers, laptop computers, other external compute devices, etc.

As shown, neckband 1205 may be coupled to eyewear device 1202 via one or more connectors. The connectors may be wired or wireless and may include electrical and/or non-electrical (e.g., structural) components. In some cases, eyewear device 1202 and neckband 1205 may operate independently without any wired or wireless connection between them. While FIG. 12 illustrates the components of eyewear device 1202 and neckband 1205 in example locations on eyewear device 1202 and neckband 1205, the components may be located elsewhere and/or distributed differently on eyewear device 1202 and/or neckband 1205. In some embodiments, the components of eyewear device 1202 and neckband 1205 may be located on one or more additional peripheral devices paired with eyewear device 1202, neckband 1205, or some combination thereof.

Pairing external devices, such as neckband 1205, with augmented-reality eyewear devices may enable the eyewear devices to achieve the form factor of a pair of glasses while still providing sufficient battery and computation power for expanded capabilities. Some or all of the battery power, computational resources, and/or additional features of augmented-reality system 1200 may be provided by a paired device or shared between a paired device and an eyewear device, thus reducing the weight, heat profile, and form factor of the eyewear device overall while still retaining desired functionality. For example, neckband 1205 may allow components that would otherwise be included on an eyewear device to be included in neckband 1205 since users may tolerate a heavier weight load on their shoulders than they would tolerate on their heads. Neckband 1205 may also have a larger surface area over which to diffuse and disperse heat to the ambient environment. Thus, neckband 1205 may allow for greater battery and computation capacity than might otherwise have been possible on a stand-alone eyewear device. Since weight carried in neckband 1205 may be less invasive to a user than weight carried in eyewear device 1202, a user may tolerate wearing a lighter eyewear device and carrying or wearing the paired device for greater lengths of time than a user would tolerate wearing a heavy standalone eyewear device, thereby enabling users to more fully incorporate artificial-reality environments into their day-to-day activities.

Neckband 1205 may be communicatively coupled with eyewear device 1202 and/or to other devices. These other devices may provide certain functions (e.g., tracking, localizing, depth mapping, processing, storage, etc.) to augmented-reality system 1200. In the embodiment of FIG. 12, neckband 1205 may include two acoustic transducers (e.g., 1220(1) and 1220(J)) that are part of the microphone array (or potentially form their own microphone subarray). Neckband 1205 may also include a controller 1225 and a power source 1235.

Acoustic transducers 1220(1) and 1220(J) of neckband 1205 may be configured to detect sound and convert the detected sound into an electronic format (analog or digital). In the embodiment of FIG. 12, acoustic transducers 1220(I) and 1220(J) may be positioned on neckband 1205, thereby increasing the distance between the neckband acoustic transducers 1220(1) and 1220(J) and other acoustic transducers 1220 positioned on eyewear device 1202. In some cases, increasing the distance between acoustic transducers 1220 of the microphone array may improve the accuracy of beamforming performed via the microphone array. For example, if a sound is detected by acoustic transducers 1220(C) and 1220(D) and the distance between acoustic transducers 1220(C) and 1220(D) is greater than, e.g., the distance between acoustic transducers 1220(D) and 1220(E), the determined source location of the detected sound may be more accurate than if the sound had been detected by acoustic transducers 1220(D) and 1220(E).

Controller 1225 of neckband 1205 may process information generated by the sensors on neckband 1205 and/or augmented-reality system 1200. For example, controller 1225 may process information from the microphone array that describes sounds detected by the microphone array. For each detected sound, controller 1225 may perform a direction-of-arrival (DOA) estimation to estimate a direction from which the detected sound arrived at the microphone array. As the microphone array detects sounds, controller 1225 may populate an audio data set with the information. In embodiments in which augmented-reality system 1200 includes an inertial measurement unit, controller 1225 may compute all inertial and spatial calculations from the IMU located on eyewear device 1202. A connector may convey information between augmented-reality system 1200 and neckband 1205 and between augmented-reality system 1200 and controller 1225. The information may be in the form of optical data, electrical data, wireless data, or any other transmittable data form. Moving the processing of information generated by augmented-reality system 1200 to neckband 1205 may reduce weight and heat in eyewear device 1202, making it more comfortable to the user.

Power source 1235 in neckband 1205 may provide power to eyewear device 1202 and/or to neckband 1205. Power source 1235 may include, without limitation, lithium ion batteries, lithium-polymer batteries, primary lithium batteries, alkaline batteries, or any other form of power storage. In some cases, power source 1235 may be a wired power source. Including power source 1235 on neckband 1205 instead of on eyewear device 1202 may help better distribute the weight and heat generated by power source 1235.

As noted, some artificial-reality systems may, instead of blending an artificial reality with actual reality, substantially replace one or more of a user's sensory perceptions of the real world with a virtual experience. One example of this type of system is a head-worn display system, such as virtual-reality system 1300 in FIG. 13, that mostly or completely covers a user's field of view. Virtual-reality system 1300 may include a front rigid body 1302 and a band 1304 shaped to fit around a user's head. Virtual-reality system 1300 may also include output audio transducers 1306(A) and 1306(B). Furthermore, while not shown in FIG. 13, front rigid body 1302 may include one or more electronic elements, including one or more electronic displays, one or more inertial measurement units (IMUs), one or more tracking emitters or detectors, and/or any other suitable device or system for creating an artificial-reality experience.

Artificial-reality systems may include a variety of types of visual feedback mechanisms. For example, display devices in augmented-reality system 1200 and/or virtual-reality system 1300 may include one or more liquid crystal displays (LCDs), light emitting diode (LED) displays, microLED displays, organic LED (OLED) displays, digital light project (DLP) micro-displays, liquid crystal on silicon (LCoS) micro-displays, and/or any other suitable type of display screen. These artificial-reality systems may include a single display screen for both eyes or may provide a display screen for each eye, which may allow for additional flexibility for varifocal adjustments or for correcting a user's refractive error. Some of these artificial-reality systems may also include optical subsystems having one or more lenses (e.g., concave or convex lenses, Fresnel lenses, adjustable liquid lenses, etc.) through which a user may view a display screen. These optical subsystems may serve a variety of purposes, including to collimate (e.g., make an object appear at a greater distance than its physical distance), to magnify (e.g., make an object appear larger than its actual size), and/or to relay (to, e.g., the viewer's eyes) light. These optical subsystems may be used in a non-pupil-forming architecture (such as a single lens configuration that directly collimates light but results in so-called pincushion distortion) and/or a pupil-forming architecture (such as a multi-lens configuration that produces so-called barrel distortion to nullify pincushion distortion).

In addition to or instead of using display screens, some of the artificial-reality systems described herein may include one or more projection systems. For example, display devices in augmented-reality system 1200 and/or virtual-reality system 1300 may include micro-LED projectors that project light (using, e.g., a waveguide) into display devices, such as clear combiner lenses that allow ambient light to pass through. The display devices may refract the projected light toward a user's pupil and may enable a user to simultaneously view both artificial-reality content and the real world. The display devices may accomplish this using any of a variety of different optical components, including waveguide components (e.g., holographic, planar, diffractive, polarized, and/or reflective waveguide elements), light-manipulation surfaces and elements (such as diffractive, reflective, and refractive elements and gratings), coupling elements, etc. Artificial-reality systems may also be configured with any other suitable type or form of image projection system, such as retinal projectors used in virtual retina displays.

The artificial-reality systems described herein may also include various types of computer vision components and subsystems. For example, augmented-reality system 1200 and/or virtual-reality system 1300 may include one or more optical sensors, such as two-dimensional (2D) or 3D cameras, structured light transmitters and detectors, time-of-flight depth sensors, single-beam or sweeping laser rangefinders, 3D LiDAR sensors, and/or any other suitable type or form of optical sensor. An artificial-reality system may process data from one or more of these sensors to identify a location of a user, to map the real world, to provide a user with context about real-world surroundings, and/or to perform a variety of other functions.

The artificial-reality systems described herein may also include one or more input and/or output audio transducers. Output audio transducers may include voice coil speakers, ribbon speakers, electrostatic speakers, piezoelectric speakers, bone conduction transducers, cartilage conduction transducers, tragus-vibration transducers, and/or any other suitable type or form of audio transducer. Similarly, input audio transducers may include condenser microphones, dynamic microphones, ribbon microphones, and/or any other type or form of input transducer. In some embodiments, a single transducer may be used for both audio input and audio output.

In some embodiments, the artificial-reality systems described herein may also include tactile (i.e., haptic) feedback systems, which may be incorporated into headwear, gloves, body suits, handheld controllers, environmental devices (e.g., chairs, floormats, etc.), and/or any other type of device or system. Haptic feedback systems may provide various types of cutaneous feedback, including vibration, force, traction, texture, and/or temperature. Haptic feedback systems may also provide various types of kinesthetic feedback, such as motion and compliance. Haptic feedback may be implemented using motors, piezoelectric actuators, fluidic systems, and/or a variety of other types of feedback mechanisms. Haptic feedback systems may be implemented independent of other artificial-reality devices, within other artificial-reality devices, and/or in conjunction with other artificial-reality devices.

By providing haptic sensations, audible content, and/or visual content, artificial-reality systems may create an entire virtual experience or enhance a user's real-world experience in a variety of contexts and environments. For instance, artificial-reality systems may assist or extend a user's perception, memory, or cognition within a particular environment. Some systems may enhance a user's interactions with other people in the real world or may enable more immersive interactions with other people in a virtual world. Artificial-reality systems may also be used for educational purposes (e.g., for teaching or training in schools, hospitals,

government organizations, military organizations, business enterprises, etc.), entertainment purposes (e.g., for playing video games, listening to music, watching video content, etc.), and/or for accessibility purposes (e.g., as hearing aids, visual aids, etc.). The embodiments disclosed herein may enable or enhance a user's artificial-reality experience in one or more of these contexts and environments and/or in other contexts and environments.

As noted, artificial-reality systems 1200 and 1300 may be used with a variety of other types of devices to provide a more compelling artificial-reality experience. These devices may be haptic interfaces with transducers that provide haptic feedback and/or that collect haptic information about a user's interaction with an environment. The artificial-reality systems disclosed herein may include various types of haptic interfaces that detect or convey various types of haptic information, including tactile feedback (e.g., feedback that a user detects via nerves in the skin, which may also be referred to as cutaneous feedback) and/or kinesthetic feedback (e.g., feedback that a user detects via receptors located in muscles, joints, and/or tendons).

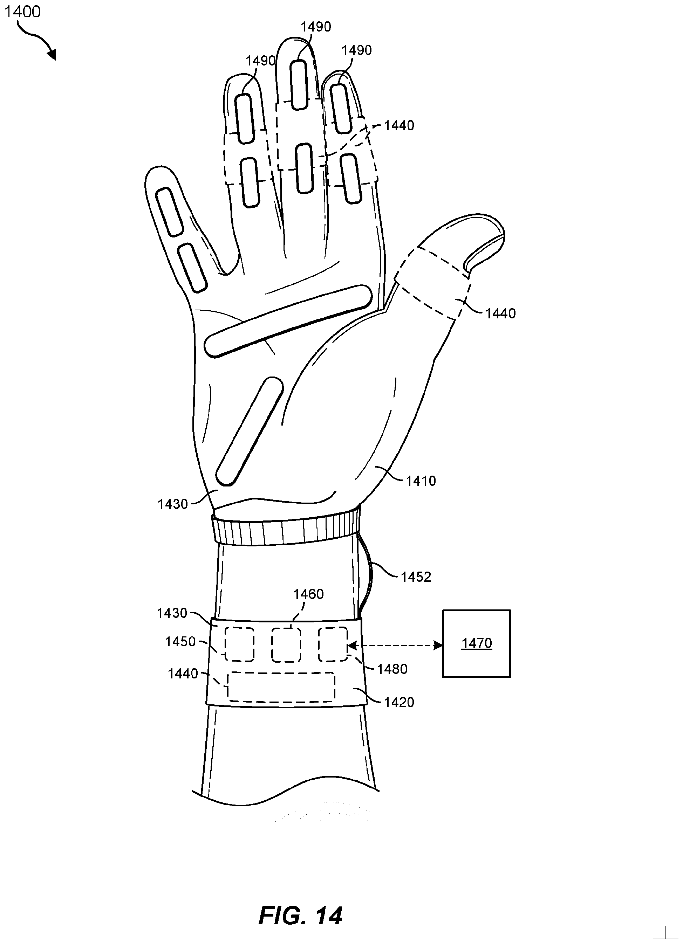

Haptic feedback may be provided by interfaces positioned within a user's environment (e.g., chairs, tables, floors, etc.) and/or interfaces on articles that may be worn or carried by a user (e.g., gloves, wristbands, etc.). As an example, FIG. 14 illustrates a vibrotactile system 1400 in the form of a wearable glove (haptic device 1410) and wristband (haptic device 1420). Haptic device 1410 and haptic device 1420 are shown as examples of wearable devices that include a flexible, wearable textile material 1430 that is shaped and configured for positioning against a user's hand and wrist, respectively. This disclosure also includes vibrotactile systems that may be shaped and configured for positioning against other human body parts, such as a finger, an arm, a head, a torso, a foot, or a leg. By way of example and not limitation, vibrotactile systems according to various embodiments of the present disclosure may also be in the form of a glove, a headband, an armband, a sleeve, a head covering, a sock, a shirt, or pants, among other possibilities. In some examples, the term "textile" may include any flexible, wearable material, including woven fabric, non-woven fabric, leather, cloth, a flexible polymer material, composite materials, etc.

One or more vibrotactile devices 1440 may be positioned at least partially within one or more corresponding pockets formed in textile material 1430 of vibrotactile system 1400. Vibrotactile devices 1440 may be positioned in locations to provide a vibrating sensation (e.g., haptic feedback) to a user of vibrotactile system 1400. For example, vibrotactile devices 1440 may be positioned against the user's finger(s), thumb, or wrist, as shown in FIG. 14. Vibrotactile devices 1440 may, in some examples, be sufficiently flexible to conform to or bend with the user's corresponding body part(s).

A power source 1450 (e.g., a battery) for applying a voltage to the vibrotactile devices 1440 for activation thereof may be electrically coupled to vibrotactile devices 1440, such as via conductive wiring 1452. In some examples, each of vibrotactile devices 1440 may be independently electrically coupled to power source 1450 for individual activation. In some embodiments, a processor 1460 may be operatively coupled to power source 1450 and configured (e.g., programmed) to control activation of vibrotactile devices 1440.

Vibrotactile system 1400 may be implemented in a variety of ways. In some examples, vibrotactile system 1400 may be a standalone system with integral subsystems and components for operation independent of other devices and systems. As another example, vibrotactile system 1400 may be configured for interaction with another device or system 1470. For example, vibrotactile system 1400 may, in some examples, include a communications interface 1480 for receiving and/or sending signals to the other device or system 1470. The other device or system 1470 may be a mobile device, a gaming console, an artificial-reality (e.g., virtual-reality, augmented-reality, mixed-reality) device, a personal computer, a tablet computer, a network device (e.g., a modem, a router, etc.), a handheld controller, etc. Communications interface 1480 may enable communications between vibrotactile system 1400 and the other device or system 1470 via a wireless (e.g., Wi-Fi, BLUETOOTH, cellular, radio, etc.) link or a wired link. If present, communications interface 1480 may be in communication with processor 1460, such as to provide a signal to processor 1460 to activate or deactivate one or more of the vibrotactile devices 1440.

Vibrotactile system 1400 may optionally include other subsystems and components, such as touch-sensitive pads 1490, pressure sensors, motion sensors, position sensors, lighting elements, and/or user interface elements (e.g., an on/off button, a vibration control element, etc.). During use, vibrotactile devices 1440 may be configured to be activated for a variety of different reasons, such as in response to the user's interaction with user interface elements, a signal from the motion or position sensors, a signal from the touch-sensitive pads 1490, a signal from the pressure sensors, a signal from the other device or system 1470, etc.

Although power source 1450, processor 1460, and communications interface 1480 are illustrated in FIG. 14 as being positioned in haptic device 1420, the present disclosure is not so limited. For example, one or more of power source 1450, processor 1460, or communications interface 1480 may be positioned within haptic device 1410 or within another wearable textile.

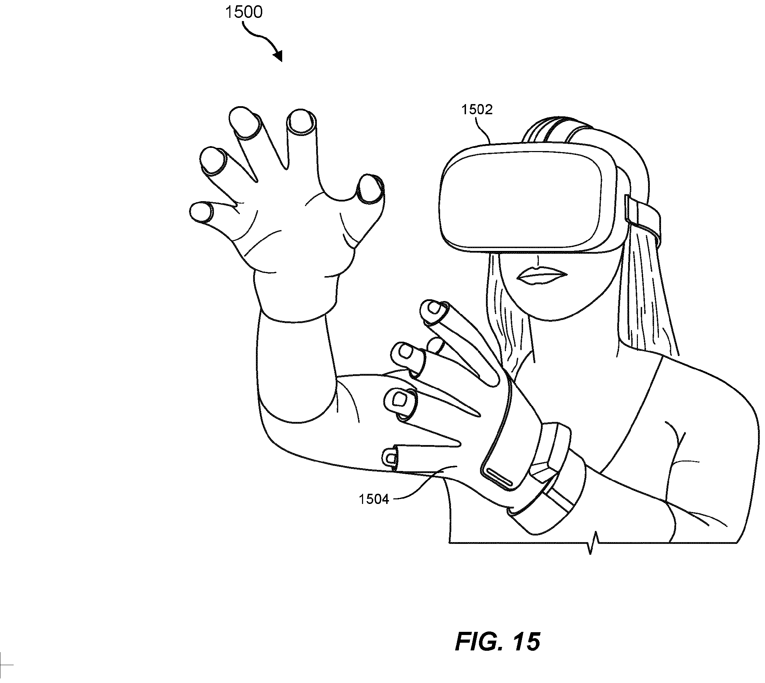

Haptic wearables, such as those shown in and described in connection with FIG. 14, may be implemented in a variety of types of artificial-reality systems and environments. FIG. 15 shows an example artificial-reality environment 1500 including one head-mounted virtual-reality display and two haptic devices (i.e., gloves), and in other embodiments any number and/or combination of these components and other components may be included in an artificial-reality system. For example, in some embodiments there may be multiple head-mounted displays each having an associated haptic device, with each head-mounted display and each haptic device communicating with the same console, portable computing device, or other computing system.

Head-mounted display 1502 generally represents any type or form of virtual-reality system, such as virtual-reality system 1300 in FIG. 13. Haptic device 1504 generally represents any type or form of wearable device, worn by a user of an artificial-reality system, that provides haptic feedback to the user to give the user the perception that he or she is physically engaging with a virtual object. In some embodiments, haptic device 1504 may provide haptic feedback by applying vibration, motion, and/or force to the user. For example, haptic device 1504 may limit or augment a user's movement. To give a specific example, haptic device 1504 may limit a user's hand from moving forward so that the user has the perception that his or her hand has come in physical contact with a virtual wall. In this specific example, one or more actuators within the haptic device may achieve the physical-movement restriction by pumping fluid into an inflatable bladder of the haptic device. In some examples, a user may also use haptic device 1504 to send action requests to a console. Examples of action requests include, without limitation, requests to start an application and/or end the application and/or requests to perform a particular action within the application.

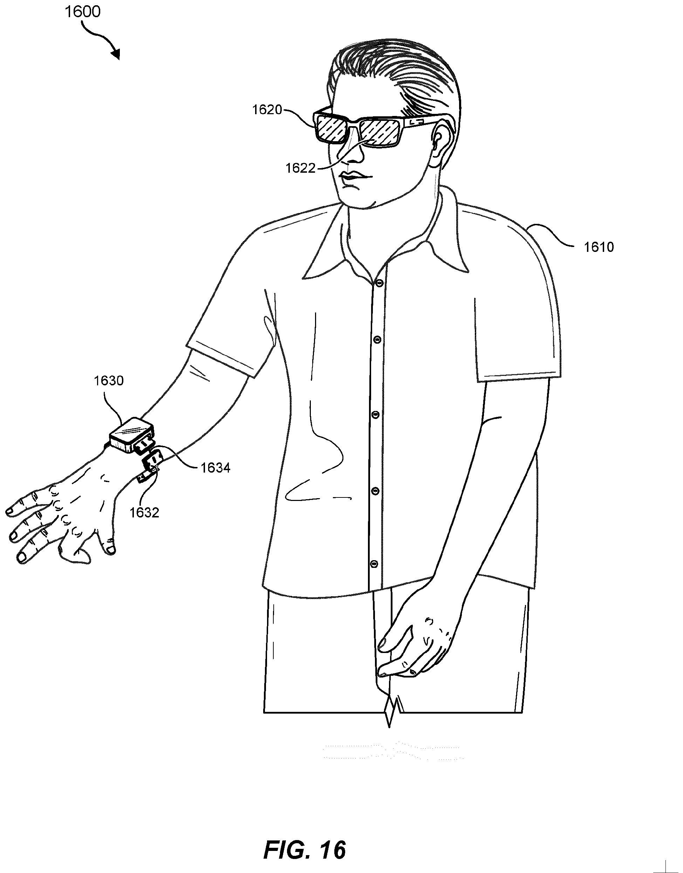

While haptic interfaces may be used with virtual-reality systems, as shown in FIG. 15, haptic interfaces may also be used with augmented-reality systems, as shown in FIG. 16. FIG. 16 is a perspective view of a user 1610 interacting with an augmented-reality system 1600. In this example, user 1610 may wear a pair of augmented-reality glasses 1620 that may have one or more displays 1622 and that are paired with a haptic device 1630. In this example, haptic device 1630 may be a wristband that includes a plurality of band elements 1632 and a tensioning mechanism 1634 that connects band elements 1632 to one another.

One or more of band elements 1632 may include any type or form of actuator suitable for providing haptic feedback. For example, one or more of band elements 1632 may be configured to provide one or more of various types of cutaneous feedback, including vibration, force, traction, texture, and/or temperature. To provide such feedback, band elements 1632 may include one or more of various types of actuators. In one example, each of band elements 1632 may include a vibrotactor (e.g., a vibrotactile actuator) configured to vibrate in unison or independently to provide one or more of various types of haptic sensations to a user. Alternatively, only a single band element or a subset of band elements may include vibrotactors.

Haptic devices 1410, 1420, 1504, and 1630 may include any suitable number and/or type of haptic transducer, sensor, and/or feedback mechanism. For example, haptic devices 1410, 1420, 1504, and 1630 may include one or more mechanical transducers, piezoelectric transducers, and/or fluidic transducers. Haptic devices 1410, 1420, 1504, and 1630 may also include various combinations of different types and forms of transducers that work together or independently to enhance a user's artificial-reality experience. In one example, each of band elements 1632 of haptic device 1630 may include a vibrotactor (e.g., a vibrotactile actuator) configured to vibrate in unison or independently to provide one or more of various types of haptic sensations to a user.

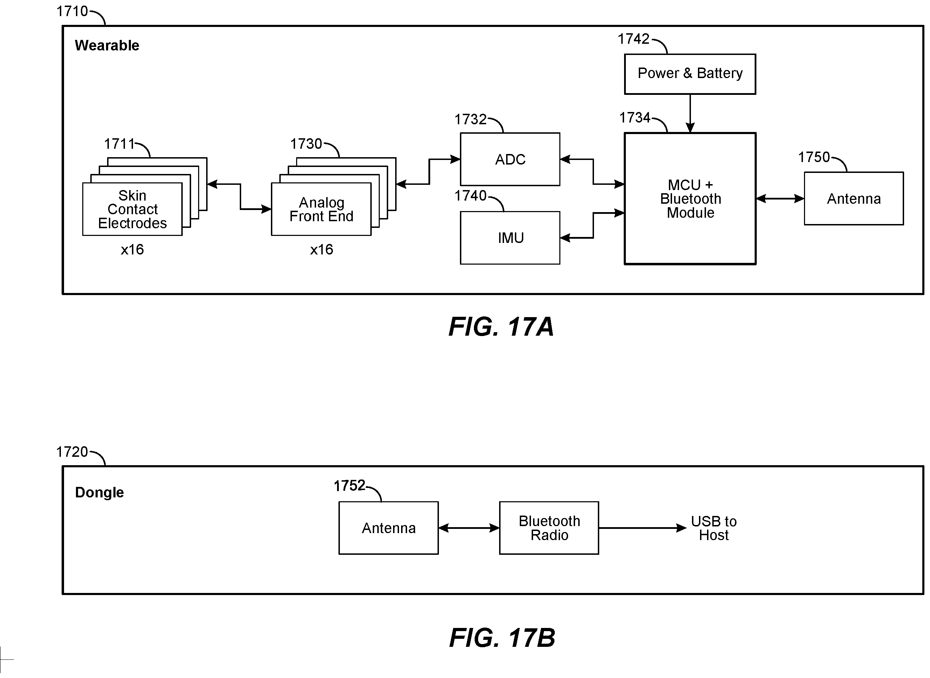

FIGS. 17A and 17B illustrate an exemplary schematic diagram with internal components of a wearable system with EMG sensors. As shown, the wearable system may include a wearable portion 1710 (FIG. 17A) and a dongle portion 1720 (FIG. 17B) in communication with the wearable portion 1710 (e.g., via BLUETOOTH or another suitable wireless communication technology). As shown in FIG. 17A, the wearable portion 1710 may include skin contact electrodes 1711 (i.e., bioelectrodes), examples of which are described in connection with FIGS. 1A and 1B (e.g., bioelectrodes 110). The output of the skin contact electrodes 1711 may be provided to analog front end 1730, which may be configured to perform analog processing (e.g., amplification, noise reduction, filtering, etc.) on the recorded signals. The processed analog signals may then be provided to analog-to-digital converter 1732, which may convert the analog signals to digital signals that can be processed by one or more computer processors. An example of a computer processor that may be used in accordance with some embodiments is microcontroller (MCU) 1734, illustrated in FIG. 17A. As shown, MCU 1734 may also include inputs from other sensors (e.g., IMU sensor 1740), and power and battery module 1742. The output of the processing performed by MCU 1734 may be provided to antenna 1750 for transmission to dongle portion 1720 shown in FIG. 17B.

Dongle portion 1720 may include antenna 1752, which may be configured to communicate with antenna 1750 included as part of wearable portion 1710. Communication between antennas 1750 and 1752 may occur using any suitable wireless technology and protocol, non-limiting examples of which include radiofrequency signaling and BLUETOOTH. As shown, the signals received by antenna 1752 of dongle portion 1720 may be provided to a host computer for further processing, display, and/or for effecting control of a particular physical or virtual object or objects.

Although the examples provided with reference to FIGS. 17A-17B are discussed in the context of interfaces with EMG sensors, the techniques described herein for reducing electromagnetic interference can also be implemented in wearable interfaces with other types of sensors including, but not limited to, mechanomyography (MMG) sensors, sonomyography (SMG) sensors, and electrical impedance tomography (EIT) sensors. The techniques described herein for reducing electromagnetic interference can also be implemented in wearable interfaces that communicate with computer hosts through wires and cables (e.g., USB cables, optical fiber cables, etc.).

In some embodiments, one or more objects (e.g., data associated with sensors, and/or activity information) of a computing system may be associated with one or more privacy settings. These objects may be stored on or otherwise associated with any suitable computing system or application, such as, for example, a social-networking system, a client system, a third-party system, a messaging application, a photo-sharing application, a biometric data acquisition application, an artificial-reality application, and/or any other suitable computing system or application.

Privacy settings (or "access settings") for an object may be stored in any suitable manner; such as, for example, in association with the object, in an index on an authorization server, in another suitable manner, or any suitable combination thereof. A privacy setting for an object may specify how the object (or particular information associated with the object) can be accessed, stored, or otherwise used (e.g., viewed, shared, modified, copied, executed, surfaced, or identified) within an application (such as an artificial-reality application). When privacy settings for an object allow a particular user or other entity to access that object, the object may be described as being "visible" with respect to that user or other entity. As an example, a user of an artificial-reality application may specify privacy settings for a user-profile page that identify a set of users that may access the artificial-reality application information on the user-profile page, thus excluding other users from accessing that information. As another example, an artificial-reality application may store privacy policies/guidelines. The privacy policies/guidelines may specify what information of users may be accessible by which entities and/or by which processes (e.g., internal research, advertising algorithms, machine-learning algorithms), thus ensuring only certain information of the user may be accessed by certain entities or processes.

In some embodiments, privacy settings for an object may specify a "blocked list" of users or other entities that should not be allowed to access certain information associated with the object. In some cases, the blocked list may include third-party entities. The blocked list may specify one or more users or entities for which an object is not visible.

Privacy settings associated with an object may specify any suitable granularity of permitted access or denial of access. As an example, access or denial of access may be specified for particular users (e.g., only me, my roommates, my boss), users within a particular degree-of-separation (e.g., friends, friends-of-friends), user groups (e.g., the gaming club, my family), user networks (e.g., employees of particular employers, students or alumni of particular university), all users ("public"), no users ("private"), users of third-party systems, particular applications (e.g., third-party applications, external websites), other suitable entities, or any suitable combination thereof. In some embodiments, different objects of the same type associated with a user may have different privacy settings. In addition, one or more default privacy settings may be set for each object of a particular object-type.

As detailed above, the computing devices and systems described and/or illustrated herein broadly represent any type or form of computing device or system capable of executing computer-readable instructions, such as those contained within the modules described herein. In their most basic configuration, these computing device(s) may each include at least one memory device and at least one physical processor.

In some examples, the term "memory device" generally refers to any type or form of volatile or non-volatile storage device or medium capable of storing data and/or computer-readable instructions. In one example, a memory device may store, load, and/or maintain one or more of the modules described herein. Examples of memory devices include, without limitation, Random Access Memory (RAM), Read Only Memory (ROM), flash memory, Hard Disk Drives (HDDs), Solid-State Drives (SSDs), optical disk drives, caches, variations or combinations of one or more of the same, or any other suitable storage memory.

In some examples, the term "physical processor" generally refers to any type or form of hardware-implemented processing unit capable of interpreting and/or executing computer-readable instructions. In one example, a physical processor may access and/or modify one or more modules stored in the above-described memory device. Examples of physical processors include, without limitation, microprocessors, microcontrollers, Central Processing Units (CPUs), Field-Programmable Gate Arrays (FPGAs) that implement softcore processors, Application-Specific Integrated Circuits (ASICs), portions of one or more of the same, variations or combinations of one or more of the same, or any other suitable physical processor.

Although illustrated as separate elements, the modules described and/or illustrated herein may represent portions of a single module or application. In addition, in certain embodiments one or more of these modules may represent one or more software applications or programs that, when executed by a computing device, may cause the computing device to perform one or more tasks. For example, one or more of the modules described and/or illustrated herein may represent modules stored and configured to run on one or more of the computing devices or systems described and/or illustrated herein. One or more of these modules may also represent all or portions of one or more special-purpose computers configured to perform one or more tasks.

In addition, one or more of the modules described herein may transform data, physical devices, and/or representations of physical devices from one form to another. For example, one or more of the modules recited herein may receive biopotential signals to be transformed, transform the biopotential signals into data indicating a user's vital characteristic(s), output a result of the transformation, use the result of the transformation to provide information to the user, and store the result of the transformation. Additionally or alternatively, one or more of the modules recited herein may transform a processor, volatile memory, non-volatile memory, and/or any other portion of a physical computing device from one form to another by executing on the computing device, storing data on the computing device, and/or otherwise interacting with the computing device.

In some embodiments, the term "computer-readable medium" generally refers to any form of device, carrier, or medium capable of storing or carrying computer-readable instructions. Examples of computer-readable media include, without limitation, transmission-type media, such as carrier waves, and non-transitory-type media, such as magnetic-storage media (e.g., hard disk drives, tape drives, and floppy disks), optical-storage media (e.g., Compact Disks (CDs), Digital Video Disks (DVDs), and BLU-RAY disks), electronic-storage media (e.g., solid-state drives and flash media), and other distribution systems.

The process parameters and sequence of the steps described and/or illustrated herein are given by way of example only and can be varied as desired. For example, while the steps illustrated and/or described herein may be shown or discussed in a particular order, these steps do not necessarily need to be performed in the order illustrated or discussed. The various exemplary methods described and/or illustrated herein may also omit one or more of the steps described or illustrated herein or include additional steps in addition to those disclosed.

The preceding description has been provided to enable others skilled in the art to best utilize various aspects of the exemplary embodiments disclosed herein. This exemplary description is not intended to be exhaustive or to be limited to any precise form disclosed. Many modifications and variations are possible without departing from the scope of the present disclosure. The embodiments disclosed herein should be considered in all respects illustrative and not restrictive. Reference should be made to the appended claims and their equivalents in determining the scope of the present disclosure.

Unless otherwise noted, the terms "connected to" and "coupled to" (and their derivatives), as used in the specification and claims, are to be construed as permitting both direct and indirect (i.e., via other elements or components) connection. In addition, the terms "a" or "an," as used in the specification and claims, are to be construed as meaning "at least one of." Finally, for ease of use, the terms "including" and "having" (and their derivatives), as used in the specification and claims, are interchangeable with and have the same meaning as the word "comprising."

## Claims

1. A biopotential measurement system comprising:
a wearable device comprising:
at least one bioelectrode positioned to abut a user's skin when the wearable device is worn by the user; and
biosignal-acquiring circuitry that captures a biosignal from the user's body via the at least one bioelectrode,
wherein measurement performance of the at least one bioelectrode is substantially optimized when a temperature of the at least one bioelectrode is at or above a threshold temperature.

2. The biopotential measurement system of any preceding claim, further comprising a temperature sensor for measuring a temperature of at least one of the at least one bioelectrode or a portion of the user's skin.

3. The biopotential measurement system of any preceding claim,
wherein the threshold temperature comprises a temperature of approximately 30 °C or more.

4. The biopotential measurement system of any preceding claim,
wherein a skin-electrode impedance of the at least one bioelectrode is approximately 0.6 MΩ or less when the temperature of the at least one bioelectrode is at or above the threshold temperature.

5. The biopotential measurement system of any preceding claim, wherein:
the wearable device is configured to extend around at least a portion of the user's arm or head; and
the at least one bioelectrode is positioned at an inner surface portion of the wearable device.

6. The biopotential measurement system of any preceding claim, further comprising an indicator that provides notification to the user of whether the bioelectrode is at or above the threshold temperature.

7. The biopotential measurement system of any preceding claim,
wherein the at least one bioelectrode comprises a metal material.

8. The biopotential measurement system of any preceding claim,
wherein the biopotential measurement system further comprises a heating subsystem that heats the at least one bioelectrode.

9. The biopotential measurement system of claim 8, and any one or more of:
a) wherein the heating subsystem preheats the at least one bioelectrode to the temperature at or above the threshold temperature when the wearable device is not worn by the user; or
b) wherein the heating subsystem is located in the wearable device; or
c) further comprising a charging device for providing power to the wearable device, wherein the heating subsystem is located in the charging device, in which case optionally wherein: the charging device comprises a charging dock; and the heating subsystem heats the at least one bioelectrode when the wearable device is disposed on the charging dock.

10. The biopotential measurement system of claim 8, wherein the heating subsystem comprises a heating element.

11. The biopotential measurement system of claim 10, and any one or more of:-
a) wherein the heating element is disposed in close proximity to the bioelectrode; or
b) wherein the heating element comprises at least one of a conductive heating element or a radiant heating element.

12. The wearable device of any preceding claim, wherein the at least one bioelectrode comprises a plurality of electrodes disposed at different locations.

13. A wearable device comprising:
at least one bioelectrode positioned to abut a user's skin when the wearable device is worn by the user;
biosignal-acquiring circuitry that captures a biosignal from the user's body via the at least one bioelectrode; and
a heating subsystem that heats the at least one bioelectrode.

14. A method comprising:
heating at least one bioelectrode of a wearable device such that a temperature of the at least one bioelectrode is at or above a threshold temperature;
positioning the at least one bioelectrode abutting a user's skin when the wearable device is worn by the user; and
capturing a biosignal from the user's body via the at least one bioelectrode.

15. The method of claim 18, wherein the threshold temperature comprises a temperature of approximately 30 °C or more, in which case optionally further comprising heating the at least one bioelectrode prior to positioning the at least one bioelectrode abutting the user's skin.
